# EUROPEAN PATENT APPLICATION

(11) **EP 4 721 803 A1**
(43) Date of publication of application: **08.04.2026**
(21) Application number: 25206751.7
(22) Date of filing: 06.10.2025
(51) Int. Cl.: A61M 25/01, A61B 18/14, A61B 90/00, A61B 18/00

(54) **ARTICULATION STATE INDICATOR FOR STEERABLE CATHETER**

(30) Priority: 07.10.2024 US 202418907781
(71) Applicant: Biosense Webster (Israel) Ltd., 2066717 Yokneam (IL)
(72) Inventor: RUBIO, Ron Dominick R., Irvine, 92618 (US); RADHAKRISHNAN, Sivaraman, Santa Clara, 95054 (US); AGNEW, William J., V, Irvine, 92618 (US); SIDDIQUI, Masood H., Irvine, 92618 (US); ABOYTES, Donald A., Irvine, 92618 (US); BANANDO, Michael D., Irvine, 92618 (US)
(74) Representative: Carpmaels & Ransford LLP

(57) **Abstract**

A guiding sheath assembly includes an elongate shaft and a control handle (910) proximal of the elongate shaft. The control handle has a longitudinal axis, and includes a control handle housing (912) and at least one shuttle (914a, 914b) configured for translation along the longitudinal axis. The guiding sheath assembly also includes at least one actuator element (921a, 921b) extending along at least one side of the elongate shaft and having a proximal end portion responsive to translation of the at least one shuttle in at least a proximal direction. The guiding sheath assembly further includes at least one deflection sensor assembly (920a, 920b) secured to the control handle housing and configured to generate one or more signals indicative of an articulation state of the elongate shaft based on a longitudinal position of the at least one shuttle.

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively applying electrical energy to cardiac tissue, it may be possible to cease or modify the propagation of unwanted electrical signals from one portion of the heart to another. Some such ablation treatments may include radio frequency (RF) ablation with alternating current (AC) electrical energy; and/or irreversible electroporation (IRE) via pulsed field direct current (DC) electrical energy. The ablation process may provide a barrier to unwanted electrical pathways by creating electrically insulative lesions or scar tissue that effectively block communication of aberrant electrical signals across the ablated tissue.

In some procedures, a catheter with one or more electrodes may be used to provide ablation within a patient. The catheter may be inserted into a major vein or artery (e.g., the femoral artery) and then advanced to position the electrodes within the heart or in a structure adjacent to the heart (e.g., the pulmonary vein). The one or more electrodes may be placed in contact with cardiac tissue or other vascular tissue and then activated with electrical energy to thereby ablate the contacted tissue (e.g., via RF energy, IRE, etc.). In some cases, the electrodes may be bipolar. In some other cases, a monopolar electrode may be used in conjunction with a ground pad or other reference electrode that is in contact with the patient. Irrigation may be used to draw heat from components of an ablation catheter; and to prevent the formation of blood clots near the tissue treatment site.

Examples of ablation catheters are described in U.S. Pat. No. 10,743,932, entitled "Integrated Ablation System using Catheter with Multiple Irrigation Lumens," issued August 18, 2020, the disclosure of which is incorporated by reference herein, in its entirety; U.S. Pat. No. 10,660,700, entitled "Irrigated Balloon Catheter with Flexible Circuit Electrode Assembly," issued May 26, 2020, the disclosure of which is incorporated by reference herein, in its entirety; U.S. Pat. No. 11,559,349, entitled "Ablation Catheter with a Flexible Printed Circuit Board," issued January 24, 2023, the disclosure of which is incorporated by reference herein, in its entirety; U.S. Pat. No. 10,702,177, entitled "Catheter with Bipole Electrode Spacer and Related Methods," issued July 7, 2020, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; and U.S. Pat. No. 9,801,585, entitled "Electrocardiogram Noise Reduction," issued October 31, 2017, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pat. No. 10,702,177, entitled "Catheter with Bipole Electrode Spacer and Related Methods," issued July 7, 2020, the disclosure of which is incorporated by reference herein, in its entirety.

Some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described, illustrated and claimed herein.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a top plan view of an example of a guiding sheath including a control handle;
FIG. 3 depicts a longitudinal cross-sectional view of the control handle of FIG. 2;
FIG. 4 depicts an exploded perspective view of the control handle of FIG. 2, with a housing removed;
FIG. 5A depicts a longitudinal, side cross-sectional view of another example of a control handle that may be used with the guiding sheath of FIG. 2 and having a push button switch for indicating an articulation state of the guiding sheath, showing a shuttle of the control handle positioned to place the guiding sheath in a neutral configuration;
FIG. 5B depicts a longitudinal, side cross-sectional view of the control handle of FIG. 5A, showing the shuttle of the control handle positioned to place the guiding sheath in a first deflected configuration;
FIG. 5C depicts a longitudinal, side cross-sectional view of the control handle of FIG. 5A, showing the shuttle of the control handle positioned to place the guiding sheath in a second deflected configuration;
FIG. 6A depicts a longitudinal, side cross-sectional view of another example of a control handle that may be used with the guiding sheath of FIG. 2 and having dual push button switches for indicating an articulation state of the guiding sheath, showing a shuttle of the control handle positioned to place the guiding sheath in a neutral configuration;
FIG. 6B depicts a longitudinal, side cross-sectional view of the control handle of FIG. 6A, showing the shuttle of the control handle positioned to place the guiding sheath in a first deflected configuration;
FIG. 6C depicts a longitudinal, side cross-sectional view of the control handle of FIG. 6A, showing the shuttle of the control handle positioned to place the guiding sheath in a second deflected configuration;
FIG. 7 depicts a perspective view of the shuttle of FIG. 6A;
FIG. 8A depicts a longitudinal, top cross-sectional view of another example of a control handle that may be used with the guiding sheath of FIG. 2 and having an optical proximity sensor for indicating an articulation state of the guiding sheath, showing a first shuttle of the control handle positioned to place the guiding sheath in a neutral configuration (with a second shuttle of the control handle omitted);
FIG. 8B depicts a longitudinal, top cross-sectional view of the control handle of FIG. 8A, showing the first shuttle of the control handle positioned to place the guiding sheath in a first deflected configuration;
FIG. 8C depicts a longitudinal, top cross-sectional view of the control handle of FIG. 8A, showing the first shuttle of the control handle positioned to place the guiding sheath in a second deflected configuration;
FIG. 9A depicts a longitudinal, top cross-sectional view of another example of a control handle that may be used with the guiding sheath of FIG. 2 and having dual optical proximity sensors for indicating an articulation state of the guiding sheath, showing shuttles of the control handle positioned to place the guiding sheath in a neutral configuration;
FIG. 9B depicts a longitudinal, top cross-sectional view of the control handle of FIG. 9A, showing the shuttles of the control handle positioned to place the guiding sheath in a first deflected configuration;
FIG. 9C depicts a longitudinal, top cross-sectional view of the control handle of FIG. 9A, showing the shuttles of the control handle positioned to place the guiding sheath in a second deflected configuration;
FIG. 10A depicts a longitudinal, top cross-sectional view of another example of a control handle that may be used with the guiding sheath of FIG. 2 and having a reed switch for indicating an articulation state of the guiding sheath, showing shuttles of the control handle positioned to place the guiding sheath in a neutral configuration;
FIG. 10B depicts a longitudinal, top cross-sectional view of the control handle of FIG. 10A, showing the shuttles of the control handle positioned to place the guiding sheath in a first deflected configuration;
FIG. 10C depicts a longitudinal, top cross-sectional view of the control handle of FIG. 10A, showing the shuttles of the control handle positioned to place the guiding sheath in a second deflected configuration;
FIG. 11A depicts a longitudinal, top cross-sectional view of another example of a control handle that may be used with the guiding sheath of FIG. 2 and having dual reed switches for indicating an articulation state of the guiding sheath, showing shuttles of the control handle positioned to place the guiding sheath in a neutral configuration;
FIG. 11B depicts a longitudinal, top cross-sectional view of the control handle of FIG. 11A, showing the shuttles of the control handle positioned to place the guiding sheath in a first deflected configuration;
FIG. 11C depicts a longitudinal, top cross-sectional view of the control handle of FIG. 11A, showing the shuttles of the control handle positioned to place the guiding sheath in a second deflected configuration;
FIG. 12A depicts a partial perspective view of another example of a control handle that may be used with the guiding sheath of FIG. 2 and having dual stop contact sensors for indicating an articulation state of the guiding sheath, showing shuttles of the control handle positioned to place the guiding sheath in a neutral configuration;
FIG. 12B depicts a partial perspective view of the control handle of FIG. 12A, showing the shuttles of the control handle positioned to place the guiding sheath in a first deflected configuration;
FIG. 12C depicts a partial perspective view of the control handle of FIG. 12A, showing the shuttles of the control handle positioned to place the guiding sheath in a second deflected configuration;
FIG. 13A depicts a longitudinal, top cross-sectional view of another example of a control handle that may be used with the guiding sheath of FIG. 2 and having a flexible sensor assembly for indicating an articulation state of the guiding sheath, showing shuttles of the control handle positioned to place the guiding sheath in a neutral configuration;
FIG. 13B depicts a longitudinal, top cross-sectional view of the control handle of FIG. 13A, showing the shuttles of the control handle positioned to place the guiding sheath in a first deflected configuration;
FIG. 13C depicts a longitudinal, top cross-sectional view of the control handle of FIG. 13A, showing the shuttles of the control handle positioned to place the guiding sheath in a second deflected configuration;
FIG. 14A depicts a schematic view of a graphical user interface that may be presented by the display of FIG. 1 based on one or more signals generated using any of the control handles of FIGS. 5A-13C when the guiding sheath is in a neutral configuration; and
FIG. 14B depicts a schematic view of a graphical user interface that may be presented by the display of FIG. 1 based on one or more signals generated using any of the control handles of FIGS. 5A-13C when the guiding sheath is in a deflected configuration.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

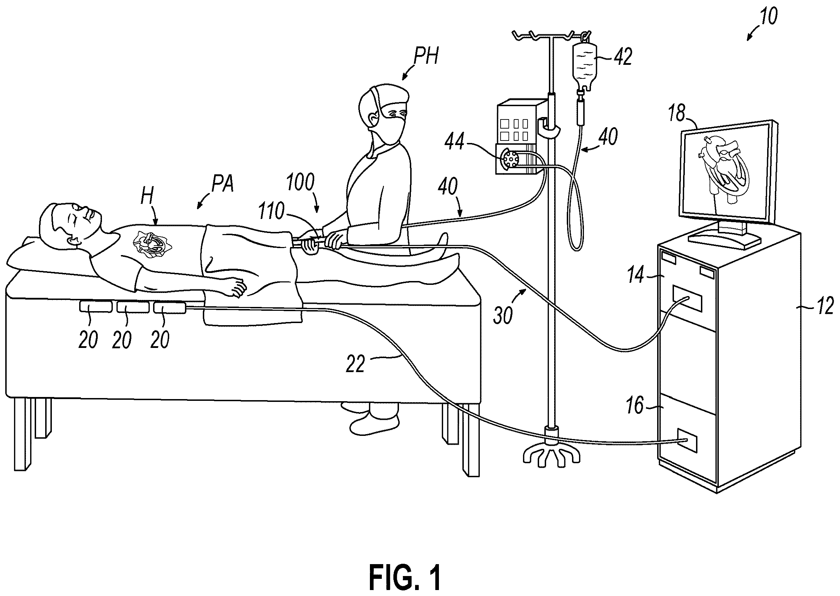

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector of a catheter (not shown) of catheter assembly (100) disposed in a patient (PA) to map potentials in tissue and/or ablate tissue in or near the heart (H) of the patient (PA).

Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Field generators (20) are merely optional.

Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via the electrodes of the end effector. Console (12) includes a processor (not shown) that processes such EP mapping signals and thereby provides EP mapping as is known in the art. In some other versions, the end effector includes other electrodes that are configured to provide EP mapping signals. In still other versions, the end effector does not provide EP mapping.

First driver module (14) of the present example is further operable to provide electrical power to the electrodes of the end effector, as will be described in greater detail below, to thereby ablate tissue. In some other versions, the end effector includes other electrodes that are configured to provide ablation. In still other versions, the end effector does not provide ablation.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from a navigation sensor assembly (not shown) in the catheter near the end effector. In such versions, the processor of console (12) is also operable to process the position indicative signals from the navigation sensor assembly to thereby determine the position of the end effector within the patient (PA). In some versions, the navigation sensor assembly includes two or more coils that are operable to generate signals that are indicative of the position and orientation of the end effector within the patient (PA). The coils are configured to generate electrical signals in response to the presence of an alternating electromagnetic field generated by field generators (20). Other components and techniques that may be used to generate real-time position data associated with the end effector may include wireless triangulation, acoustic tracking, optical tracking, inertial tracking, and the like. While the navigation sensor assembly is shown as being disposed in the distal end of the catheter, the navigation sensor assembly may instead be positioned in the end effector. Alternatively, the catheter and the end effector may lack a navigation sensor assembly.

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the navigation sensor assembly of the end effector. For instance, as the end effector of the catheter moves within the patient (PA), the corresponding position data from the navigation sensor assembly may cause the processor of console (12) to update the patient anatomy views in display (18) in real time to depict the regions of patient anatomy around the end effector as the end effector moves within the patient (PA). Moreover, the processor of console (12) may drive display (18) to show locations of aberrant conductive tissue sites, as detected via electrophysiological (EP) mapping with the end effector or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of the end effector on the images of the patient's anatomy, such as by superimposing an illuminated dot, a crosshair, a graphical representation of the end effector, or some other form of visual indication. Such a superimposed visual indication may also move within the images of the patient anatomy on display (18) in real time as the physician moves the end effector within the patient (PA), thereby providing real-time visual feedback to the operator about the position of the end effector within the patient (PA) as the end effector moves within the patient (PA). The images provided through display (18) may thus effectively provide a video tracking the position of the end effector within a patient (PA), without necessarily having any optical instrumentation (i.e., cameras) viewing the end effector. In the same view, display (18) may simultaneously visually indicate the locations of aberrant conductive tissue sites detected through EP mapping. The physician (PH) may thus view display (18) to observe the real time positioning of the end effector in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). Such irrigation fluid may be expelled through openings (not shown) of the end effector. Such irrigation may be provided in any suitable fashion as will be apparent to those skilled in the art in view of the teachings herein.

### II. Example of a Guiding Sheath Assembly

In some procedures, the physician (PH) may wish to introduce a catheter into the patient (PA) via a delivery sheath (also referred to as a guiding sheath). In some such procedures, the delivery sheath may be inserted into the patient (PA) (e.g., via the leg or groin of the patient (PA)); and then be advanced along a vein or artery to reach a position in or near the heart (H). Once the delivery sheath is suitably positioned in the patient (PA), the physician (PH) may then advance an end effector and catheter into the delivery sheath. Once the end effector is suitably positioned near the targeted structure, the delivery sheath may be retracted relative to the end effector (or the end effector may be advanced relative to the delivery sheath). In some instances, the end effector may then be expanded by inflating a balloon of the end effector to bring the electrodes into contact with the tissue of the targeted structure. In addition, or in the alternative, one or more resilient features of the end effector may urge end effector from a non-expanded state to an expanded state. Once the end effector is in the expanded state, the physician (PH) may then operate catheter assembly (100) to provide EP mapping, ablation, or any other kind of operations in or near the heart (H) of the patient (PA). The end effector may then be collapsed to the non-expanded configuration by deflating the balloon, and/or otherwise contracting the end effector, for retraction through the delivery sheath.

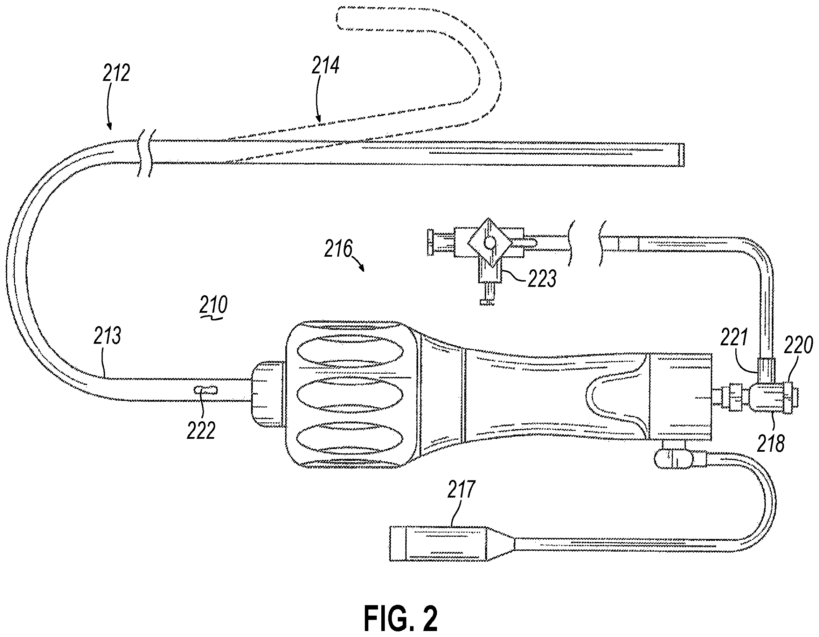

FIGS. 2-4 show an example of a guiding sheath assembly (210) that may be used in such procedures. Guiding sheath assembly (210) includes an elongate flexible sheath (212), and a control handle (216) proximal of sheath (212). Sheath (212) includes a proximal section (213) and a distal deflection section (214). Control handle (216) may be connected to an electrical connector (217) for transmitting electrical signals, as sensed by one or more ring electrodes carried on sheath (212), including, for example, deflection section (214). Also attached to control handle (216), as shown in FIG. 2, is a hemostatic valve (218) adapted to receive a catheter (not shown) that can be advanced through a center lumen (222) of guiding sheath assembly (210) (FIG. 2). Hemostatic valve (218) also has side port (221) terminating in a luer hub, such as a two-way stop cock (223), for connection to one or more fluid sources (not shown) for providing fluid into and through lumen (222) of guiding sheath assembly (210).

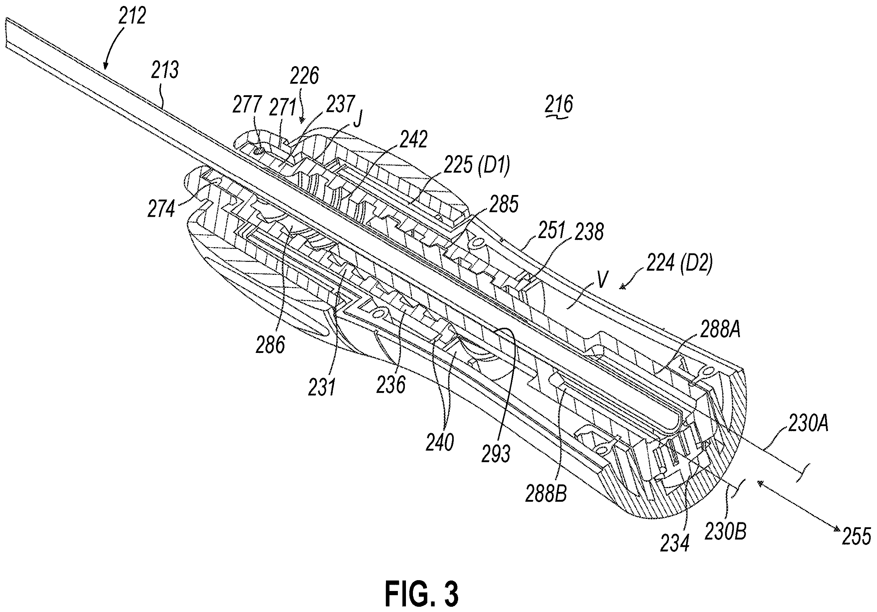

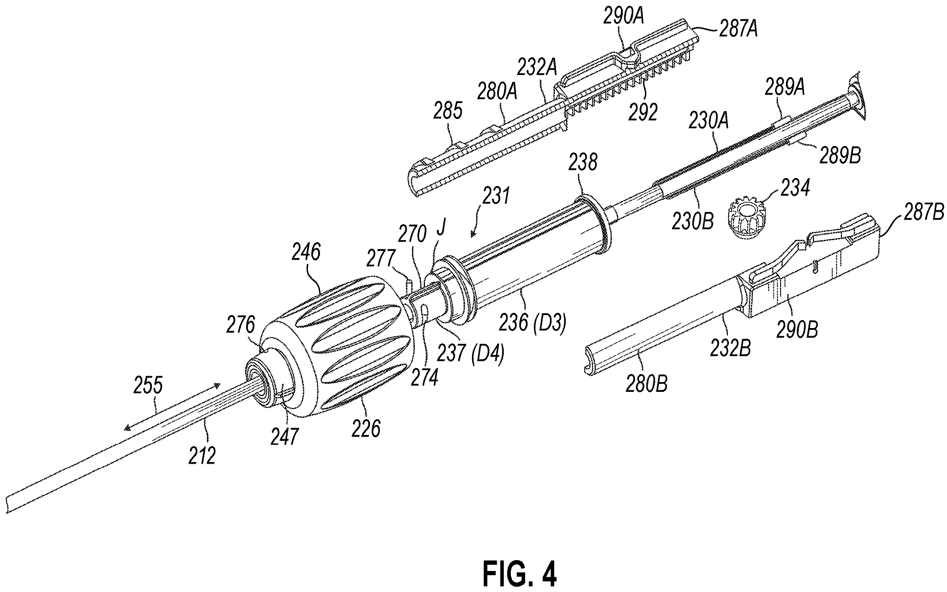

As shown in FIGS. 3 and 4, control handle (216) includes an elongate, generally cylindrical main body (224) with a narrower distal portion or stem (225), and a distal rotational control knob (226) mounted on distal stem (225). Main body (224) has an outer shell-half member formed to define an interior volume (V) and whose edges (251) meet along a longitudinal seam. Distal stem (225) of main body (224) has a smaller outer diameter (D1) compared to the outer diameter (D2) of a proximal portion of main body (224). Control knob (226) is configured for rotation by a user's thumb and forefinger when the user is grasping main body (224) of control handle (216). To enable deflection of deflection section (214) of guiding sheath (212) via first and second puller wires (230A, 230B), control handle (216) includes in its interior volume (V) a rotatable shaft (231), first and second shuttles (232A, 232B), and a pinion (234). Rotatable shaft (231) is responsive to control knob (226) in driving first shuttle (232A) to move linearly along a longitudinal axis (255) in a first direction, and pinion (234) couples second shuttle (232B) to first shuttle (232A) such that second shuttle (232B) moves linearly along the longitudinal axis in a second direction opposition to the first direction. With proximal ends of the first and second puller wires (230A, 230B) anchored, or at least coupled, to first and second shuttles (232A, 232B), respectively, such coupled and opposite translational movement of the first and second shuttles actuate first and second puller wires (230A, 230B) for bi-directional deflection of deflection section (214) of guiding sheath (212).

The rotatable shaft (231) has a main proximal section (236) with an outer diameter (D3), a shorter distal section (237) with an outer diameter (D4), and a step junction (J) between proximal and distal sections (236, 237). Rotatable shaft (231) is connected and affixed at its proximal end to main body (224) by a proximal outer circumferential lip (238) that engages with an inner circumferential slot defined between circumferential flanges (240) formed in the interior volume (V) of main body (224). Rotatable shaft (231) is hollow having an interior passage (242). Passage (242) is threaded and has a diameter to accommodate both the guiding sheath (212) and shuttles (232A, 232B) circumferentially surrounding guiding sheath (212), as discussed below in further detail.

Control knob (226), which is mounted on distal stem (225) of main body (224) of control handle (216) and rotatable shaft (231), has a main proximal portion (246) and a short distal end portion (247). Control knob (226) is generally cylindrical with a longitudinal hollow interior that extends through its entire length.

To rotationally couple rotatable shaft (231) to control knob (226) (for common rotational movement of rotatable shaft (231) and control knob (226)), an outer surface of the distal section of shaft (231) has a longitudinal ridge (270) (FIG. 4) that is received in and engages with a corresponding longitudinal recess (271) (FIG. 3) formed on an inner surface of control knob (226). To translationally affix control knob (226) to rotatable shaft (231) (for common translational movement of control knob (226) and rotatable shaft (231)) and hence main body (224), the outer surface of shaft (231) also has one or more linear slots (274) oriented perpendicularly to the longitudinal axis of rotatable shaft (231). Each slot (274) is aligned with a respective hole (276) (FIG. 4) formed through a side of distal end portion (247) of control knob (226), so that a respective pin (277) may be inserted into hole (276) and slot (274) to couple control knob (226) and rotatable shaft (231) for common translational movement.

As shown in FIG. 4, shuttles (232A, 232B) have a similar construction to each other, with the understanding that each is generally a mirror image of the other, although first shuttle (232A) is driven by rotatable shaft (231) and second shuttle (232B) is driven by first shuttle (232A) via pinion (234) situated between them. Each shuttle (232A, 232B) has a respective elongate body having a distal portion (280A, 280B) with a C-shaped end cross-section, and a respective proximal rack portion (290A, 290B) with a respective plurality of teeth (292) arranged longitudinally. First and second shuttles (232A, 232B) are arranged to face each other and engage pinion (234) such that distal portions (280A, 280B) together can form a cylindrical form with an outer circumferential surface that fits within threaded passage (242), and an inner circumferential surface that defines a passage (293) for guiding sheath (212) to pass through. Rack portion (290A, 290B) of each shuttle (232A, 232B) face each other with pinion (234) in between so that teeth (292) of each rack portion (290A, 290B) can engage with teeth of pinion (234) which is mounted for rotation about an axis perpendicular to the longitudinal axis (255) of control handle (216).

With reference to FIGS. 3 and 4, an outer surface of distal portion (280A) of first shuttle (232A) is configured with an external or male threaded surface (285). An inner circumferential surface of rotatable shaft (231) is configured with an internal or female threaded surface (286) (FIG. 3) which receives male threaded surface (285) of first shuttle (232A) for coupling first shuttle (232A) and rotatable shaft (231) in converting rotational movement of rotatable shaft (231) into translation movement of first shuttle (232A). Accordingly, as a user rotates control knob (226) in a first direction, rotatable shaft (231) which is rotationally coupled to control knob (226) (for common rotational movement) via longitudinal ridge (270) also rotates. With rotatable shaft (231) rotationally and translationally locked to control knob (226) (for common rotational and common translational movement) via longitudinal ridge (270) and one or more pins (277), rotation of shaft (231) drives first shuttle (232A) to translate along the longitudinal axis in a first direction (for example, proximally). As first shuttle (232A) translates, its teeth (292) drive pinion (234) to rotate in a first direction (for example, clockwise), which in turn drives second shuttle (232B) to translate along the longitudinal axis (255) in a second direction opposite of the first direction (for example, distally). So arranged, male and female threaded surfaces (285, 286) convert rotational movement of control knob (226) into linear movement of shuttles (232A, 232B). With proximal ends of first and second puller wires (230A, 230B) anchored, coupled or otherwise responsive to first and second shuttles (232A, 232B), respectively, linear and opposite movements of shuttles (232A, 232B) actuate puller wires (230A, 230B) for bi-directional deflection of deflection section (214) of guiding sheath (212). In the example shown, the proximal ends of puller wires (230A, 230B) are coupled to rack portions (290A, 290B) of shuttles (232A, 232B), respectively. Thus, when one puller wire (230A, 230B) is drawn proximally under tension by its respective shuttle (232A, 232B), the other puller wire (230A, 230B) is simultaneously released from tension by its respective shuttle (232A, 232B) moving distally.

As shown in FIG. 3, a proximal end segment of each puller wire (230A, 230B) extends outside of sheath (212), in a respective longitudinal channel (288A, 288B) formed in the proximal rack portion (290A, 290B) of each shuttle (232A, 232B). As shown in FIG. 4, a stop (289A, 289B), for example, a hypotube, is affixed to the proximal end of each puller wire (230A, 230B), and the stop is positioned proximal of a proximal end (287A, 287B) of the respective rack portion (290A, 290B) so that each rack portion (290A, 290B) can push or otherwise act on the respective stop (289A, 289B) to draw the respective puller wire (230A, 230B) proximally when the respective shuttle (232A, 232B) is moved proximally. When a shuttle (232A, 232B) is moved distally, the proximal end of the respective rack portion (290A, 290B) comes out of contact with the respective stop (289A, 289B), releasing the respective puller wire (230A, 230B) from tension. It is understood that each stop (289A, 289B) may also be embedded or otherwise anchored to the respective rack portion (290A, 290B) or any part of the respective shuttle (232A, 232B) to effect deflection of sheath (212).

Because first and second shuttles (232A, 232B) move in opposite directions along the longitudinal axis (255), an initial positioning of shuttles (232A, 232B) relative to each other and to passage (242) is made during assembly of control handle (216). For example, as shown in FIG. 3, each shuttle (232A, 232B) is positioned in passage (242) of rotatable shaft (231) such that shuttles (232A, 232B) are even with each other along the longitudinal axis (255), and each shuttle (232A, 232B) has a distal end positioned generally at a mid-point along passage (242) so that each shuttle (232A, 232B) has sufficient room to move correspondingly proximally or distally within rotatable shaft (231). Stops (289A, 289B) may be positioned relative to shuttles (232A, 232B) such that there is minimal or even tension exerted on each puller wire (230A, 230B) for a generally neutral guiding sheath with little, if any, deflection. So arranged, shuttles (232A, 232B) adopt a "neutral" or initial configuration from which the user may evenly deflect guiding sheath (212) bidirectionally.

By way of further example only, guiding sheath assembly (210) may be constructed and operable in accordance with at least some of the teachings of U.S. Pat. No. 10,653,860, entitled "Steerable Guiding Sheath with Rack and Pinion Deflection Mechanism," issued May 19, 2020, the disclosure of which is incorporated by reference herein. In some versions, guiding sheath assembly (210) is configured and operable like the CARTO VIZIGO^{®} bi-directional delivery sheath by Biosense Webster, Inc. of Irvine, California.

### IV. Examples of Control Handles with Articulation State Indicator

In some procedures, it may be desirable to provide the operator with a real time indication of an articulation (e.g., deflection) state of guiding sheath (212) (and/or a catheter disposed therein). For example, it may be desirable for the operator to confirm that guiding sheath (212) is in the neutral configuration before retracting guiding sheath (212) from the patient's heart (H) (e.g., from the fossa ovalis of the patient's heart (H) and/or from a transseptal hole in the patient's heart (H)) in order to avoid inflicting trauma to the patient's heart (H) that might otherwise be caused by retracting guiding sheath (212) while in a deflected state. However, during some procedures, the operator may wish to continuously view display (18) to continuously observe the real time positioning of the end effector, catheter, and/or guiding sheath (212) in relation to mapped aberrant conductive tissue sites and/or in relation to images of the adjacent anatomical structures in the patient (PA). Thus, it may be desirable to provide the operator with the real time indication of the articulation state of guiding sheath (212) via display (18), to allow the operator to observe the real time indication of the articulation state of guiding sheath (212) substantially simultaneously with observing the real time positioning of the end effector, catheter, and/or guiding sheath (212). In this regard, it will be appreciated that providing the operator with the real time indication of the articulation state of guiding sheath (212) via display (18) may allow the operator to observe the real time indication of the articulation state of guiding sheath (212) without requiring the operator to divert the operator's attention away from display (18).

### A. Example of Control Handle with Single Push Button Switch

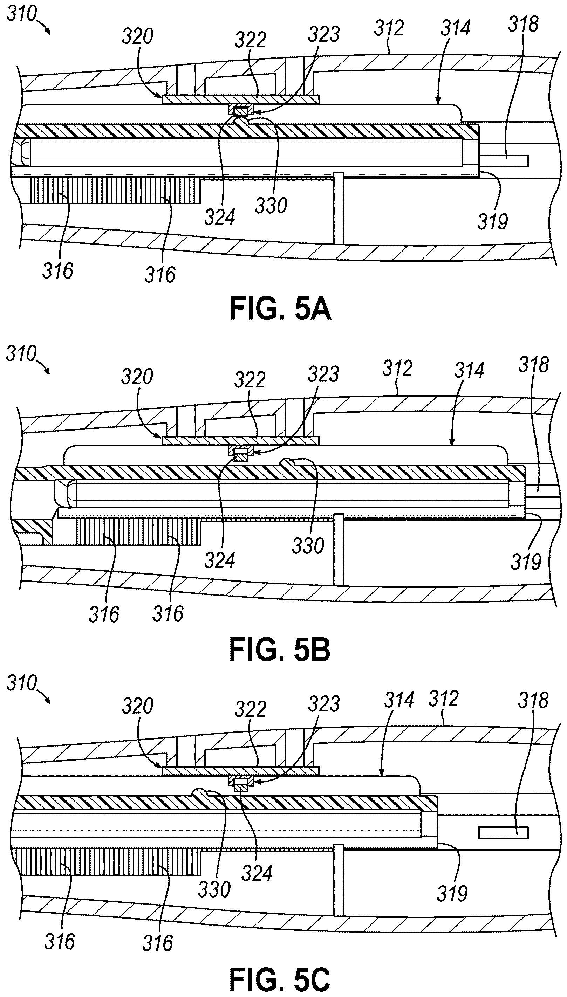

FIGS. 5A-5C show an example of a control handle (310) that may be incorporated into guiding sheath assembly (210) in place of control handle (216), and that may provide at least some, if not all, of the features and functionalities described above. Control handle (310) may be similar to control handle (216) described above, except as otherwise described below. In this regard, control handle (310) of the present example includes an elongate, generally cylindrical main body (312) similar to main body (224) described above, and a distal rotational control knob (not shown), such as distal rotational control knob (226) described above, mounted on a distal stem of main body (312). As shown, control handle (310) also includes a pair of shuttles (314) (one shown) similar to shuttles (232A, 232B) described above. A rotatable shaft (not shown), such as rotatable shaft (231) described above, is configured to drive longitudinal translation of one shuttle (314) in response to rotation of the control knob; and a pinion (not shown), such as pinion (234) described above, couples shuttles (314) to each other via respective teeth (316) of shuttles (314) to thereby drive longitudinal translation of the other shuttle (314) in an opposite direction. Stops (also referred to as ferrules) (318) (one shown) similar to stops (289A, 289B) described above are affixed to the proximal ends of respective actuator elements (not shown), such as puller wires (230A, 230B) described above or any other suitable actuator elements (e.g., bands, rods, etc.), and are each positioned proximal of a proximal end (319) of a corresponding shuttle (314) so that each shuttle (314) can push or otherwise act on the respective stop (318) to draw the respective puller wire proximally when the respective shuttle (314) is moved proximally from a neutral position (FIG. 5A) toward a proximal position (FIG. 5B); when a shuttle (314) is moved distally from the proximal position (FIG. 5B) toward the neutral position (FIG. 5A), the respective stop (318) may move distally with the shuttle (314) as tension is relieved in the respective puller wire; when a shuttle (314) is moved distally from the neutral position (FIG. 5A) toward a distal position (FIG. 5C), the proximal end (319) of the shuttle (314) comes out of contact with the respective stop (318), releasing the respective puller wire from tension, such that the respective stop (318) may remain stationary at its own neutral position. Thus, control handle (310) may provide bi-directional deflection of deflection section (214) of guiding sheath (212) in a manner similar to that described above in connection with control handle (216).

Control handle (310) of the present example further includes a deflection sensor assembly (320) configured to generate one or more signals indicative of an articulation state of guiding sheath (212). In the example shown, deflection sensor assembly (320) includes a printed circuit board (PCB) (322) fixedly secured to main body (312), and a tactile switch (323) including a push button (324) movably mounted on PCB (322) such that push button (324) is actuatable from an unactuated state (FIGS. 5B-5C) to an actuated state (FIG. 5A). In this regard, one shuttle (314) (e.g., the shuttle (314) that is driven directly by the rotatable shaft) includes an actuating member in the form of a protrusion (330), and push button (324) is disposed along the translation path of protrusion (330) such that protrusion (330) is configured to selectively actuate push button (324) by selectively engaging push button (324).

More particularly, protrusion (330) of the present example is disposed at a predetermined position along a length of shuttle (314) such that protrusion (330) is configured to align (e.g., vertically) with and engage push button (324) when shuttles (314) are even with each other in the longitudinal direction to place guiding sheath (212) in the neutral configuration; and such that protrusion (330) is configured to misalign (e.g., vertically) from and disengage push button (324) when shuttles (314) are moved proximally and distally relative to each other to place guiding sheath (212) in a deflected configuration. Therefore, the one or more signals generated by deflection sensor assembly (320) may indicate whether guiding sheath (212) is in a neutral or deflected configuration. In some cases, the engagement and/or disengagement of protrusion (330) with push button (324) may generate tactile and/or audible feedback (e.g., a "click" sound) indicative of the articulation state of guiding sheath (212).

Deflection sensor assembly (320) may be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assembly (320). In such versions, the processor of console (12) may be operable to process the articulation indicative signals from deflection sensor assembly (320) to thereby determine the current articulation state of guiding sheath (212). For instance, the processor of console (12) may be operable to determine that guiding sheath (212) is currently in the neutral configuration in response to receiving a signal from deflection sensor assembly (320) indicating that push button (324) is currently engaged by protrusion (330); and may be operable to determine that guiding sheath (212) is currently in a deflected configuration in response to not receiving a signal from deflection sensor assembly (320) (indicating that push button (324) is currently disengaged from protrusion (330)). The processor of console (12) may further be operable to provide a visual indication of the articulation state of guiding sheath (212) in real time via display (18), as described in greater detail below.

In an example of a method of use, guiding sheath (212) may initially be in the neutral configuration, such that protrusion (330) aligns with and engages push button (324) as shown in FIG. 5A, and the operator may observe a real time indication that guiding sheath (212) is in the neutral configuration on display (18). Guiding sheath (212) may subsequently be articulated to a first deflected configuration, such that protrusion (330) is proximal of and disengaged from push button (324) as shown in FIG. 5B, and the operator may observe a real time indication that guiding sheath (212) is deflected on display (18). In addition, or alternatively, guiding sheath (212) may be articulated to a second deflected configuration, such that protrusion (330) is distal of and disengaged from push button (324) as shown in FIG. 5C, and the operator may observe a real time indication that guiding sheath (212) is deflected on display (18). In some instances, guiding sheath (212) may eventually be returned to the neutral configuration, such that protrusion (330) aligns with and engages push button (324) as shown in FIG. 5A, and the operator may observe a real time indication that guiding sheath (212) is in the neutral configuration on display (18), before retracting guiding sheath (212) from the patient's heart (H).

### B. Example of Control Handle with Dual Push Button Switches

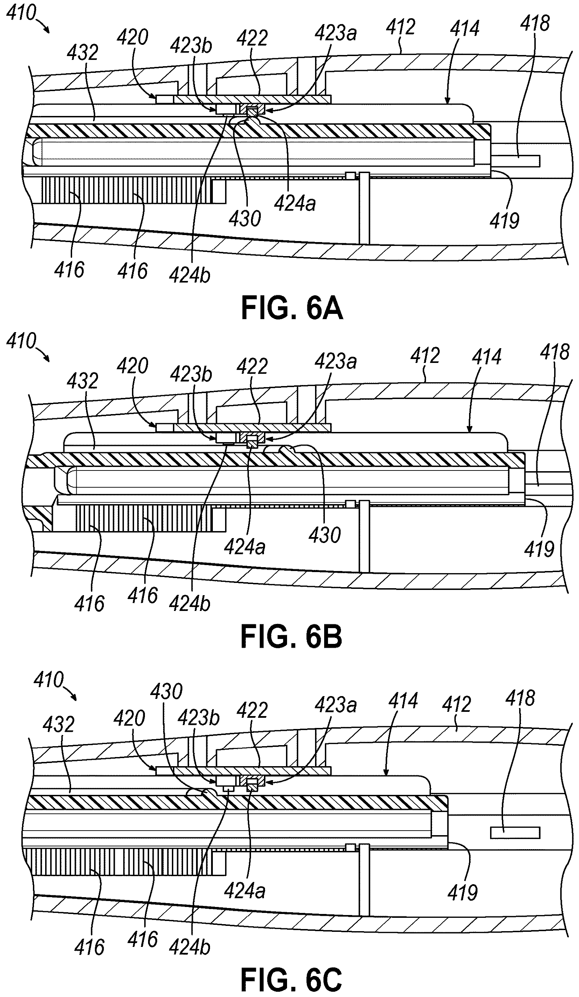

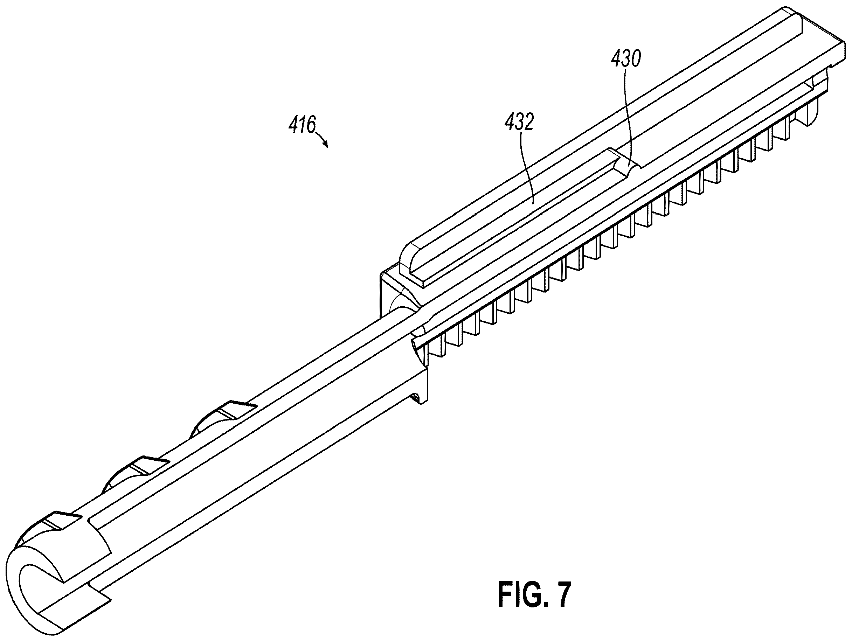

FIGS. 6A-7 show an example of a control handle (410) that may be incorporated into guiding sheath assembly (210) in place of control handle (216), and that may provide at least some, if not all, of the features and functionalities described above. Control handle (410) may be similar to control handle (216) described above, except as otherwise described below. In this regard, control handle (410) of the present example includes an elongate, generally cylindrical main body (412) similar to main body (224) described above, and a distal rotational control knob (not shown), such as distal rotational control knob (226) described above, mounted on a distal stem of main body (412). As shown, control handle (410) also includes a pair of shuttles (414) (one shown) similar to shuttles (232A, 232B) described above. A rotatable shaft (not shown), such as rotatable shaft (231) described above, is configured to drive longitudinal translation of one shuttle (414) in response to rotation of the control knob; and a pinion (not shown), such as pinion (234) described above, couples shuttles (414) to each other via respective teeth (416) of shuttles (414) to thereby drive longitudinal translation of the other shuttle (414) in an opposite direction. Stops (also referred to as ferrules) (418) (one shown) similar to stops (289A, 289B) described above are affixed to the proximal ends of respective actuator elements (not shown), such as puller wires (230A, 230B) described above or any other suitable actuator elements (e.g., bands, rods, etc.), and are each positioned proximal of a proximal end (419) of a corresponding shuttle (414) so that each shuttle (414) can push or otherwise act on the respective stop (418) to draw the respective puller wire proximally when the respective shuttle (414) is moved proximally from a neutral position (FIG. 6A) toward a proximal position (FIG. 6B); when a shuttle (414) is moved distally from the proximal position (FIG. 6B) toward the neutral position (FIG. 6A), the respective stop (418) may move distally with the shuttle (414) as tension is relieved in the respective puller wire; when a shuttle (414) is moved distally from the neutral position (FIG. 6A) toward a distal position (FIG. 6C), the proximal end (419) of the shuttle (414) comes out of contact with the respective stop (418), releasing the respective puller wire from tension, such that the respective stop (418) may remain stationary at its own neutral position. Thus, control handle (410) may provide bi-directional deflection of deflection section (214) of guiding sheath (212) in a manner similar to that described above in connection with control handle (216).

Control handle (410) of the present example further includes a deflection sensor assembly (420) configured to generate one or more signals indicative of an articulation state of guiding sheath (212). In the example shown, deflection sensor assembly (420) includes a printed circuit board (PCB) (422) fixedly secured to main body (412), and first and second laterally-adjacent tactile switches (423a, 423b) including first and second laterally-adjacent push buttons (424a, 424b) movably mounted on PCB (422) such that first push button (424a) is actuatable from an unactuated state (FIGS. 6B-6C) to an actuated state (FIG. 6A), and such that second push button (424b) is actuatable from an unactuated state (FIG. 6C) to an actuated states (FIGS. 6A-6B). In this regard, one shuttle (414) (e.g., the shuttle (414) that is driven directly by the rotatable shaft) includes a first actuating member in the form of a protrusion (430) and a second actuating member in the form of an elongate ridge (432); and first push button (424a) is disposed along the translation path of protrusion (430) such that protrusion (430) is configured to selectively actuate first push button (424a) by selectively engaging push button (424a), and second push button (424b) is disposed along the translation path of elongate ridge (432) such that elongate ridge (432) is configured to selectively actuate second push button (424b) by selectively engaging second push button (424b).

More particularly, protrusion (430) of the present example is disposed at a predetermined position along a length of shuttle (414) such that protrusion (430) is configured to align (e.g., vertically) with and engage first push button (424a) when shuttles (414) are even with each other in the longitudinal direction to place guiding sheath (212) in the neutral configuration; and such that protrusion (430) is configured to misalign (e.g., vertically) from and disengage first push button (424a) when shuttles (414) are moved proximally and distally relative to each other to place guiding sheath (212) in a deflected configuration. In some cases, the engagement and/or disengagement of protrusion (430) with first push button (424a) may generate tactile and/or audible feedback (e.g., a "click" sound) indicative of the articulation state of guiding sheath (212).

Moreover, elongate ridge (432) of the present example is disposed at a predetermined position along a length of shuttle (414) such that at least a portion of elongate ridge (432) is configured to align (e.g., vertically) with and engage second push button (424b) when shuttles (414) are even with each other in the longitudinal direction to place guiding sheath (212) in the neutral configuration, and when a first one of shuttles (414) is moved proximally and a second one of shuttles (414) is moved distally to place guiding sheath (212) in a deflected configuration in which guiding sheath is deflected in a first direction (e.g., rightward); and such that elongate ridge (432) is configured to misalign (e.g., vertically) from and disengage second push button (424b) when the first one of shuttles (414) is moved distally and the second one of shuttles (414) is moved proximally to place guiding sheath (212) in a deflected configuration in which guiding sheath is deflected in a second direction (e.g., leftward). Therefore, in addition to indicating whether guiding sheath (212) is in a neutral or deflected configuration, the one or more signals generated by deflection sensor assembly (420) may indicate a direction of deflection when guiding sheath (212) is in a deflected configuration. In some cases, the engagement and/or disengagement of elongate ridge (432) with second push button (424b) may generate tactile and/or audible feedback (e.g., a "click" sound) indicative of the articulation state of guiding sheath (212).

Deflection sensor assembly (420) may be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assembly (420). In such versions, the processor of console (12) may be operable to process the articulation indicative signals from deflection sensor assembly (420) to thereby determine the current articulation state of guiding sheath (212). For instance, the processor of console (12) may be operable to determine that guiding sheath (212) is currently in the neutral configuration in response to receiving a signal from deflection sensor assembly (420) indicating that push buttons (424a, 424b) are currently engaged by protrusion (430) and elongate ridge (432), respectively; and may be operable to determine that guiding sheath (212) is currently in a deflected configuration in which guiding sheath is deflected in the first direction (e.g., rightward) in response to receiving a signal from deflection sensor assembly (420) indicating that first push button (424a) is currently disengaged from protrusion (430) and that second push button (424b) is currently engaged by elongate ridge (432); and may be operable to determine that guiding sheath (212) is currently in a deflected configuration in which guiding sheath is deflected in the second direction (e.g., leftward) in response to not receiving a signal from deflection sensor assembly (420) (indicating that push buttons (424a, 424b) are currently disengaged from protrusion (430) and elongate ridge (432), respectively). The processor of console (12) may further be operable to provide a visual indication of the articulation state of guiding sheath (212) in real time via display (18), as described in greater detail below.

In an example of a method of use, guiding sheath (212) may initially be in the neutral configuration, such that protrusion (430) aligns with and engages first push button (424a) and such that at least a portion of elongate ridge (432) aligns with and engages second push button (424b) as shown in FIG. 6A, and the operator may observe a real time indication that guiding sheath (212) is in the neutral configuration on display (18). Guiding sheath (212) may subsequently be articulated to a first deflected configuration in which guiding sheath (212) is deflected in the first direction (e.g., rightward), such that protrusion (430) is proximal of and disengaged from first push button (424a) and such that at least a portion of elongate ridge (432) aligns with and engages second push button (424b) as shown in FIG. 6B, and the operator may observe a real time indication that guiding sheath (212) is deflected in the first direction on display (18). In addition, or alternatively, guiding sheath (212) may be articulated to a second deflected configuration in which guiding sheath (212) is deflected in the second direction (e.g., leftward), such that protrusion (430) is distal of and disengaged from first push button (424a) and such that elongate ridge (432) is distal of and disengaged from second push button (424b) as shown in FIG. 6C, and the operator may observe a real time indication that guiding sheath (212) is deflected in the second direction on display (18). In some instances, guiding sheath (212) may eventually be returned to the neutral configuration, such that protrusion (430) aligns with and engages first push button (424a) and such that at least a portion of elongate ridge (432) aligns with and engages second push button (424b) as shown in FIG. 6A, and the operator may observe a real time indication that guiding sheath (212) is in the neutral configuration on display (18), before retracting guiding sheath (212) from the patient's heart (H).

### C. Example of Control Handle with Single Optical Proximity Sensor

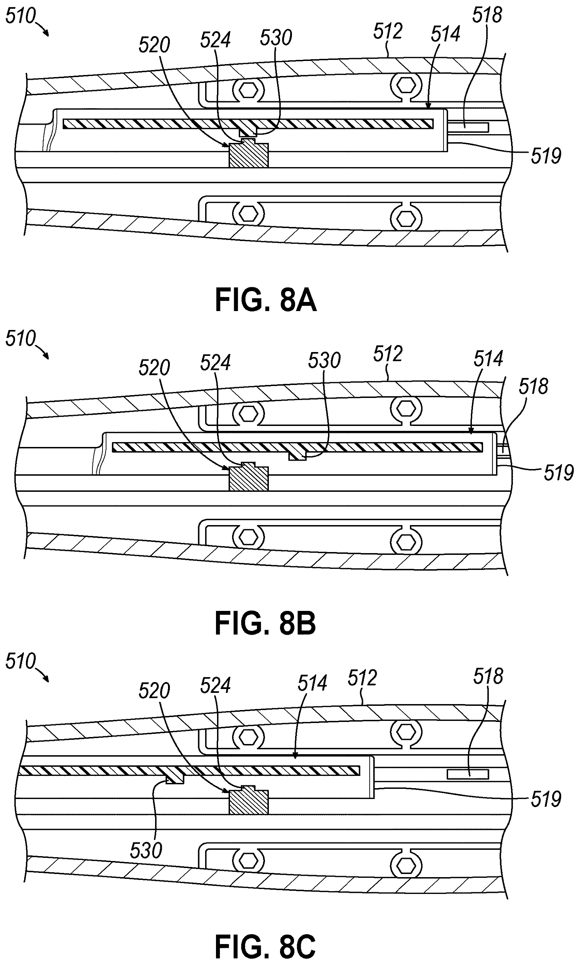

FIGS. 8A-8C show an example of a control handle (510) that may be incorporated into guiding sheath assembly (210) in place of control handle (216), and that may provide at least some, if not all, of the features and functionalities described above. Control handle (510) may be similar to control handle (216) described above, except as otherwise described below. In this regard, control handle (510) of the present example includes an elongate, generally cylindrical main body (512) similar to main body (224) described above, and a distal rotational control knob (not shown), such as distal rotational control knob (226) described above, mounted on a distal stem of main body (512). As shown, control handle (510) also includes a pair of shuttles (514) (one shown) similar to shuttles (232A, 232B) described above. A rotatable shaft (not shown), such as rotatable shaft (231) described above, is configured to drive longitudinal translation of one shuttle (514) in response to rotation of the control knob; and a pinion (not shown), such as pinion (234) described above, couples shuttles (514) to each other via respective teeth (not shown) of shuttles (514) to thereby drive longitudinal translation of the other shuttle (514) in an opposite direction. Stops (also referred to as ferrules) (518) (one shown) similar to stops (289A, 289B) described above are affixed to the proximal ends of respective actuator elements (not shown), such as puller wires (230A, 230B) described above or any other suitable actuator elements (e.g., bands, rods, etc.), and are each positioned proximal of a proximal end (519) of a corresponding shuttle (514) so that each shuttle (514) can push or otherwise act on the respective stop (518) to draw the respective puller wire proximally when the respective shuttle (514) is moved proximally from a neutral position (FIG. 8A) toward a proximal position (FIG. 8B); when a shuttle (514) is moved distally from the proximal position (FIG. 8B) toward the neutral position (FIG. 8A), the respective stop (518) may move distally with the shuttle (514) as tension is relieved in the respective puller wire; when a shuttle (514) is moved distally from the neutral position (FIG. 8A) toward a distal position (FIG. 8C), the proximal end (519) of the shuttle (514) comes out of contact with the respective stop (518), releasing the respective puller wire from tension, such that the respective stop (518) may remain stationary at its own neutral position. Thus, control handle (510) may provide bi-directional deflection of deflection section (214) of guiding sheath (212) in a manner similar to that described above in connection with control handle (216).

Control handle (510) of the present example further includes a deflection sensor assembly (520) configured to generate one or more signals indicative of an articulation state of guiding sheath (212). In the example shown, deflection sensor assembly (520) includes a proximity sensor in the form of an optical proximity sensor (524) fixedly secured to main body (512). In this regard, one shuttle (514) (e.g., the shuttle (514) that is driven directly by the rotatable shaft) includes a marker member in the form of a protrusion (530), and the field of view of optical proximity sensor (524) intersects with (e.g., is perpendicular to) the translation path of protrusion (530) such that protrusion (530) is configured to selectively trigger optical proximity sensor (524) by selectively crossing the field of view of optical proximity sensor (524).

More particularly, protrusion (530) of the present example is disposed at a predetermined position along a length of shuttle (514) such that protrusion (530) is configured to align (e.g., laterally) with and trigger optical proximity sensor (524) when shuttles (514) are even with each other in the longitudinal direction to place guiding sheath (212) in the neutral configuration; and such that protrusion (530) is configured to misalign (e.g., laterally) from and be outside the field of view of optical proximity sensor (524) when shuttles (514) are moved proximally and distally relative to each other to place guiding sheath (212) in a deflected configuration. Therefore, the one or more signals generated by deflection sensor assembly (520) may indicate whether guiding sheath (212) is in a neutral or deflected configuration; and may be generated without requiring physical contact between deflection sensor assembly (520) and either shuttle (514).

Deflection sensor assembly (520) may be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assembly (520). In such versions, the processor of console (12) may be operable to process the articulation indicative signals from deflection sensor assembly (520) to thereby determine the current articulation state of guiding sheath (212). For instance, the processor of console (12) may be operable to determine that guiding sheath (212) is currently in the neutral configuration in response to receiving a signal from deflection sensor assembly (520) indicating that optical proximity sensor (524) is currently triggered by protrusion (530); and may be operable to determine that guiding sheath (212) is currently in a deflected configuration in response to not receiving a signal from deflection sensor assembly (520) (indicating that optical proximity sensor (524) is currently not triggered by protrusion (530)). The processor of console (12) may further be operable to provide a visual indication of the articulation state of guiding sheath (212) in real time via display (18), as described in greater detail below.

In an example of a method of use, guiding sheath (212) may initially be in the neutral configuration, such that protrusion (530) aligns with and triggers optical proximity sensor (524) as shown in FIG. 8A, and the operator may observe a real time indication that guiding sheath (212) is in the neutral configuration on display (18). Guiding sheath (212) may subsequently be articulated to a first deflected configuration, such that protrusion (530) is proximal of and outside the field of view of optical proximity sensor (524) as shown in FIG. 8B, and the operator may observe a real time indication that guiding sheath (212) is deflected on display (18). In addition, or alternatively, guiding sheath (212) may be articulated to a second deflected configuration, such that protrusion (530) is distal of and outside the field of view of optical proximity sensor (524) as shown in FIG. 8C, and the operator may observe a real time indication that guiding sheath (212) is deflected on display (18). In some instances, guiding sheath (212) may eventually be returned to the neutral configuration, such that protrusion (530) aligns with and triggers optical proximity sensor (524) as shown in FIG. 8A, and the operator may observe a real time indication that guiding sheath (212) is in the neutral configuration on display (18), before retracting guiding sheath (212) from the patient's heart (H).

### D. Example of Control Handle with Dual Optical Proximity Sensors

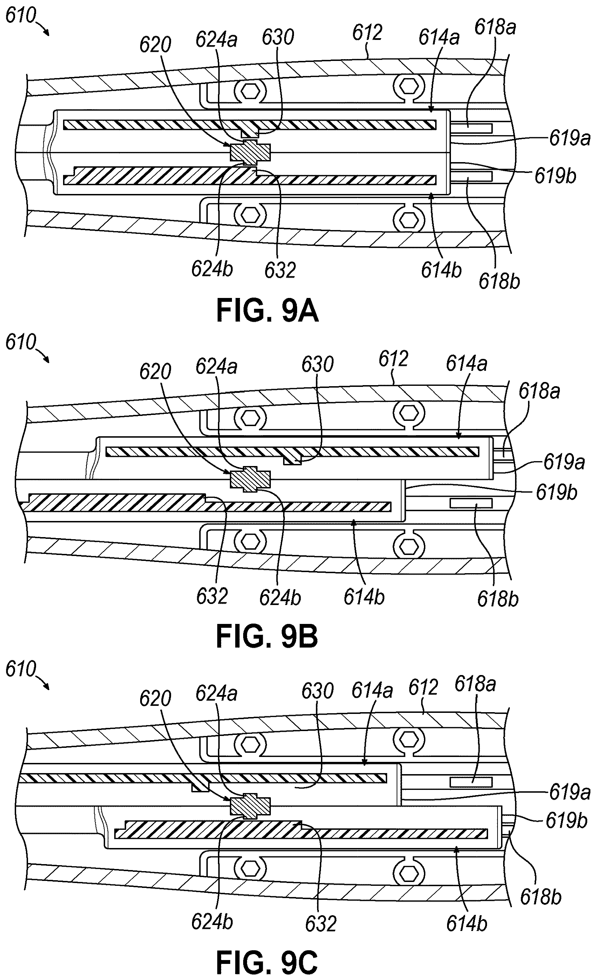

FIGS. 9A-9C show an example of a control handle (610) that may be incorporated into guiding sheath assembly (210) in place of control handle (216), and that may provide at least some, if not all, of the features and functionalities described above. Control handle (610) may be similar to control handle (216) described above, except as otherwise described below. In this regard, control handle (610) of the present example includes an elongate, generally cylindrical main body (612) similar to main body (224) described above, and a distal rotational control knob (not shown), such as distal rotational control knob (226) described above, mounted on a distal stem of main body (612). As shown, control handle (610) also includes a pair of shuttles (614a, 614b) similar to shuttles (232A, 232B) described above. A rotatable shaft (not shown), such as rotatable shaft (231) described above, is configured to drive longitudinal translation of first shuttle (614a) in response to rotation of the control knob; and a pinion (not shown), such as pinion (234) described above, couples shuttles (614a, 614b) to each other via respective teeth (not shown) of shuttles (614a, 614b) to thereby drive longitudinal translation of second shuttle (614b) in an opposite direction. Stops (also referred to as ferrules) (618a, 618b) similar to stops (289A, 289B) described above are affixed to the proximal ends of respective actuator elements (not shown), such as puller wires (230A, 230B) described above or any other suitable actuator elements (e.g., bands, rods, etc.), and are each positioned proximal of a proximal end (619a, 619b) of a corresponding shuttle (614a, 614b) so that each shuttle (614a, 614b) can push or otherwise act on the respective stop (618a, 618b) to draw the respective puller wire proximally when the respective shuttle (614a, 614b) is moved proximally from a neutral position (e.g., both shuttles (614a, 614b) in FIG. 9A) toward a proximal position (e.g., first shuttle (614a) in FIG. 9B, second shuttle (614b) in FIG. 9C); when a shuttle (614a, 614b) is moved distally from the proximal position (e.g., first shuttle (614a) in FIG. 9B, second shuttle (614b) in FIG. 9C) toward the neutral position (e.g., both shuttles (614a, 614b) in FIG. 9A), the respective stop (618a, 618b) may move distally with the shuttle (614a, 614b) as tension is relieved in the respective puller wire; when a shuttle (614a, 614b) is moved distally from the neutral position (e.g., both shuttles (614a, 614b) in FIG. 9A) toward a distal position (e.g., first shuttle (614a) in FIG. 9C, second shuttle (614b) in FIG. 9B), the proximal end (619a, 619b) of the shuttle (614a, 614b) comes out of contact with the respective stop (618a, 618b), releasing the respective puller wire from tension, such that the respective stop (618a, 618b) may remain stationary at its own neutral position. Thus, control handle (610) may provide bi-directional deflection of deflection section (214) of guiding sheath (212) in a manner similar to that described above in connection with control handle (216).

Control handle (610) of the present example further includes a deflection sensor assembly (620) configured to generate one or more signals indicative of an articulation state of guiding sheath (212). In the example shown, deflection sensor assembly (620) includes first and second laterally-opposing proximity sensors in the form of optical proximity sensors (624a, 624b) fixedly secured to main body (612). In this regard, first shuttle (614a) includes a first marker member in the form of a protrusion (630), and second shuttle (614b) includes a second marker member in the form of an elongate ridge (632); and the field of view of first optical proximity sensor (624a) intersects with (e.g., is perpendicular to) the translation path of first protrusion (630a) such that first protrusion (630a) is configured to selectively trigger first optical proximity sensor (624a) by selectively crossing the field of view of first optical proximity sensor (624a), and the field of view of second optical proximity sensor (624b) intersects with (e.g., is perpendicular to) the translation path of elongate ridge (632) such that elongate ridge (632) is configured to selectively trigger second optical proximity sensor (624b) by selectively crossing the field of view of second optical proximity sensor (624b).

More particularly, protrusion (630) of the present example is disposed at a predetermined position along a length of first shuttle (614a) such that protrusion (630) is configured to align (e.g., laterally) with and trigger first optical proximity sensor (624a) when shuttles (614a, 614b) are even with each other in the longitudinal direction to place guiding sheath (212) in the neutral configuration; and such that protrusion (630) is configured to misalign (e.g., laterally) from and be outside the field of view of first optical proximity sensor (624a) when shuttles (614a, 614b) are moved proximally and distally relative to each other to place guiding sheath (212) in a deflected configuration.

Moreover, elongate ridge (632) of the present example is disposed at a predetermined position along a length of second shuttle (614b) such that at least a portion of elongate ridge (632) is configured to align (e.g., laterally) with and trigger second optical proximity sensor (624b) when shuttles (614a, 614b) are even with each other in the longitudinal direction to place guiding sheath (212) in the neutral configuration, and when a second shuttle (614b) is moved proximally and first shuttle (614a) is moved distally to place guiding sheath (212) in a deflected configuration in which guiding sheath is deflected in a first direction (e.g., leftward); and such that elongate ridge (632) is configured to misalign (e.g., laterally) from and be outside the field of view of second optical proximity sensor (624b) when second shuttle (614b) is moved distally and first shuttle (614a) is moved proximally to place guiding sheath (212) in a deflected configuration in which guiding sheath is deflected in a second direction (e.g., rightward). Therefore, in addition to indicating whether guiding sheath (212) is in a neutral or deflected configuration, the one or more signals generated by deflection sensor assembly (620) may indicate a direction of deflection when guiding sheath (212) is in a deflected configuration; and may be generated without requiring physical contact between deflection sensor assembly (620) and either shuttle (614a, 614b).

Deflection sensor assembly (620) may be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assembly (620). In such versions, the processor of console (12) may be operable to process the articulation indicative signals from deflection sensor assembly (620) to thereby determine the current articulation state of guiding sheath (212). For instance, the processor of console (12) may be operable to determine that guiding sheath (212) is currently in the neutral configuration in response to receiving a signal from deflection sensor assembly (620) indicating that optical proximity sensors (624a, 624b) are currently triggered by protrusion (630) and elongate ridge (632), respectively; and may be operable to determine that guiding sheath (212) is currently in a deflected configuration in which guiding sheath is deflected in the first direction (e.g., leftward) in response to receiving a signal from deflection sensor assembly (620) indicating that first optical proximity sensor (624a) is currently not triggered by protrusion (630) and that second optical proximity sensor (624b) is currently triggered by elongate ridge (632); and may be operable to determine that guiding sheath (212) is currently in a deflected configuration in which guiding sheath is deflected in the second direction (e.g., rightward) in response to not receiving a signal from deflection sensor assembly (620) (indicating that optical proximity sensors (624a, 624b) are currently not triggered by protrusion (630) and elongate ridge (632), respectively). The processor of console (12) may further be operable to provide a visual indication of the articulation state of guiding sheath (212) in real time via display (18), as described in greater detail below.

In an example of a method of use, guiding sheath (212) may initially be in the neutral configuration, such that protrusion (630) aligns with and triggers first optical proximity sensor (624a) and such that at least a portion of elongate ridge (632) aligns with and triggers second optical proximity sensor (624b) as shown in FIG. 9A, and the operator may observe a real time indication that guiding sheath (212) is in the neutral configuration on display (18). Guiding sheath (212) may subsequently be articulated to a first deflected configuration in which guiding sheath (212) is deflected in the first direction (e.g., leftward), such that protrusion (630) is distal of and outside the field of view of first optical proximity sensor (624a) and such that at least a portion of elongate ridge (632) aligns with and triggers second optical proximity sensor (624b) as shown in FIG. 9C, and the operator may observe a real time indication that guiding sheath (212) is deflected in the first direction on display (18). In addition, or alternatively, guiding sheath (212) may be articulated to a second deflected configuration in which guiding sheath (212) is deflected in the second direction (e.g., rightward), such that protrusion (630) is proximal of and outside the field of view of first optical proximity sensor (624a) and such that elongate ridge (632) is distal of and disengaged from second optical proximity sensor (624b) as shown in FIG. 9B, and the operator may observe a real time indication that guiding sheath (212) is deflected in the second direction on display (18). In some instances, guiding sheath (212) may eventually be returned to the neutral configuration, such that protrusion (630) aligns with and triggers first optical proximity sensor (624a) and such that at least a portion of elongate ridge (632) aligns with and triggers second optical proximity sensor (624b) as shown in FIG. 9A, and the operator may observe a real time indication that guiding sheath (212) is in the neutral configuration on display (18), before retracting guiding sheath (212) from the patient's heart (H).

### E. Example of Control Handle with Single Reed Switch

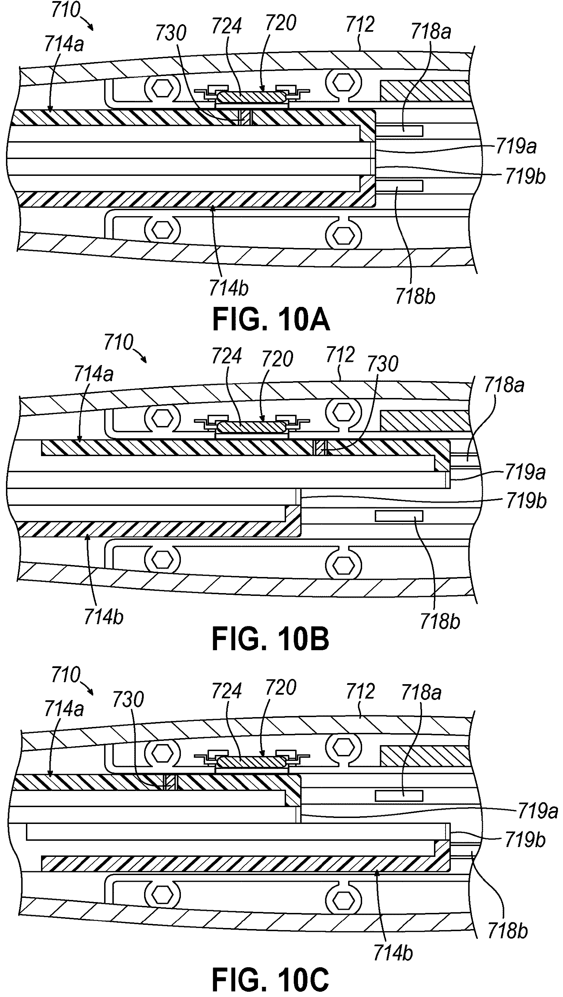

FIGS. 10A-10C show an example of a control handle (710) that may be incorporated into guiding sheath assembly (210) in place of control handle (216), and that may provide at least some, if not all, of the features and functionalities described above. Control handle (710) may be similar to control handle (216) described above, except as otherwise described below. In this regard, control handle (710) of the present example includes an elongate, generally cylindrical main body (712) similar to main body (224) described above, and a distal rotational control knob (not shown), such as distal rotational control knob (226) described above, mounted on a distal stem of main body (712). As shown, control handle (710) also includes a pair of shuttles (714a, 714b) similar to shuttles (232A, 232B) described above. A rotatable shaft (not shown), such as rotatable shaft (231) described above, is configured to drive longitudinal translation of one shuttle (714a, 714b) in response to rotation of the control knob; and a pinion (not shown), such as pinion (234) described above, couples shuttles (714a, 714b) to each other via respective teeth (not shown) of shuttles (714a, 714b) to thereby drive longitudinal translation of the other shuttle (714a, 714b) in an opposite direction. Stops (also referred to as ferrules) (718a, 718b) similar to stops (289A, 289B) described above are affixed to the proximal ends of respective actuator elements (not shown), such as puller wires (230A, 230B) described above or any other suitable actuator elements (e.g., bands, rods, etc.), and are each positioned proximal of a proximal end (719a, 719b) of a corresponding shuttle (714a, 714b) so that each shuttle (714a, 714b) can push or otherwise act on the respective stop (718a, 718b) to draw the respective puller wire proximally when the respective shuttle (714a, 714b) is moved proximally from a neutral position (e.g., both shuttles (714a, 714b) in FIG. 10A) toward a proximal position (e.g., first shuttle (714a) in FIG. 10B, second shuttle (714b) in FIG. 10C); when a shuttle (714a, 714b) is moved distally from the proximal position (e.g., first shuttle (714a) in FIG. 10B, second shuttle (714b) in FIG. 10C) toward the neutral position (e.g., both shuttles (714a, 714b) in FIG. 10A), the respective stop (718a, 718b) may move distally with the shuttle (714a, 714b) as tension is relieved in the respective puller wire; when a shuttle (714a, 714b) is moved distally from the neutral position (e.g., both shuttles (714a, 714b) in FIG. 10A) toward a distal position (e.g., first shuttle (714a) in FIG. 10C, second shuttle (714b) in FIG. 10B), the proximal end (719a, 719b) of the shuttle (714a, 714b) comes out of contact with the respective stop (718a, 718b), releasing the respective puller wire from tension, such that the respective stop (718a, 718b) may remain stationary at its own neutral position. Thus, control handle (710) may provide bi-directional deflection of deflection section (214) of guiding sheath (212) in a manner similar to that described above in connection with control handle (216).

Control handle (710) of the present example further includes a deflection sensor assembly (720) configured to generate one or more signals indicative of an articulation state of guiding sheath (212). In the example shown, deflection sensor assembly (720) includes a proximity sensor in the form of a reed switch (724) fixedly secured to main body (712). In this regard, first shuttle (714a) includes a magnetic member in the form of an embedded permanent magnet (730), and the activation zone of reed switch (724) intersects with (e.g., is perpendicular to) the translation path of magnet (730) such that magnet (730) is configured to selectively trigger reed switch (724) by selectively crossing the activation zone of reed switch (724).

More particularly, magnet (730) of the present example is disposed at a predetermined position along a length of first shuttle (714a) such that magnet (730) is configured to trigger reed switch (724) when shuttles (714a, 714b) are even with each other in the longitudinal direction to place guiding sheath (212) in the neutral configuration; and such that magnet (730) is configured to be outside the activation zone of reed switch (724) when shuttles (714a, 714b) are moved proximally and distally relative to each other to place guiding sheath (212) in a deflected configuration. Therefore, the one or more signals generated by deflection sensor assembly (720) may indicate whether guiding sheath (212) is in a neutral or deflected configuration; and may be generated using relatively low current and without requiring physical contact between deflection sensor assembly (720) and either shuttle (714). In some versions, magnet (730) may be oriented perpendicular to the longitudinal axis that shuttles (714a, 714b) translate along to improve the resolution of reed switch (724).

Deflection sensor assembly (720) may be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assembly (720). In such versions, the processor of console (12) may be operable to process the articulation indicative signals from deflection sensor assembly (720) to thereby determine the current articulation state of guiding sheath (212). For instance, the processor of console (12) may be operable to determine that guiding sheath (212) is currently in the neutral configuration in response to receiving a signal from deflection sensor assembly (720) indicating that reed switch (724) is currently triggered by magnet (730); and may be operable to determine that guiding sheath (212) is currently in a deflected configuration in response to not receiving a signal from deflection sensor assembly (720) (indicating that reed switch (724) is currently not triggered by magnet (730)). The processor of console (12) may further be operable to provide a visual indication of the articulation state of guiding sheath (212) in real time via display (18), as described in greater detail below.

In an example of a method of use, guiding sheath (212) may initially be in the neutral configuration, such that magnet (730) triggers reed switch (724) as shown in FIG. 10A, and the operator may observe a real time indication that guiding sheath (212) is in the neutral configuration on display (18). Guiding sheath (212) may subsequently be articulated to a first deflected configuration, such that magnet (730) is proximal of and outside the activation zone of reed switch (724) as shown in FIG. 10B, and the operator may observe a real time indication that guiding sheath (212) is deflected on display (18). In addition, or alternatively, guiding sheath (212) may be articulated to a second deflected configuration, such that magnet (730) is distal of and outside the activation zone of reed switch (724) as shown in FIG. 10C, and the operator may observe a real time indication that guiding sheath (212) is deflected on display (18). In some instances, guiding sheath (212) may eventually be returned to the neutral configuration, such that magnet (730) triggers reed switch (724) as shown in FIG. 10A, and the operator may observe a real time indication that guiding sheath (212) is in the neutral configuration on display (18), before retracting guiding sheath (212) from the patient's heart (H).

### F. Example of Control Handle with Dual Reed Switches

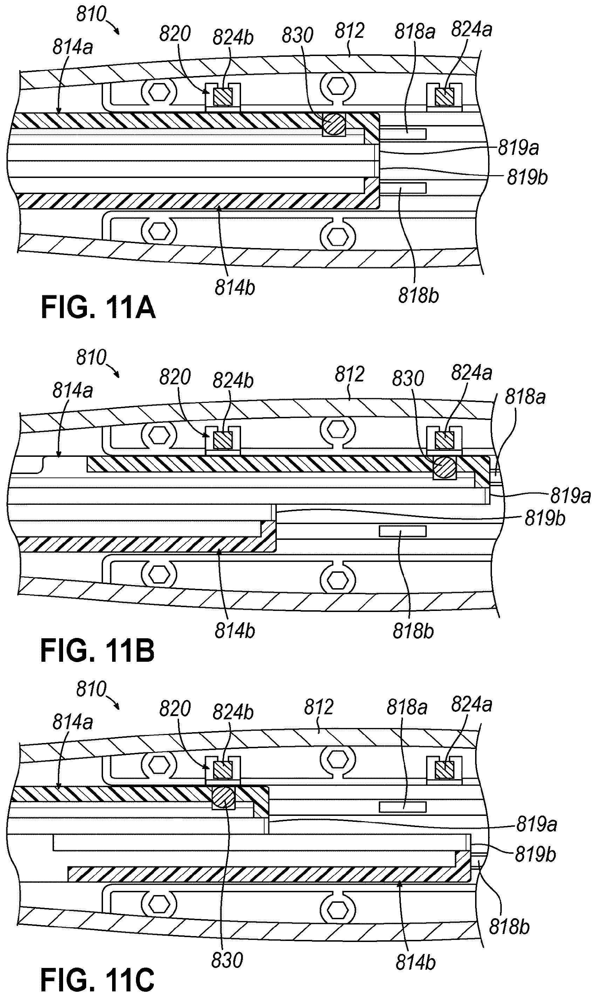

FIGS. 11A-11C show an example of a control handle (810) that may be incorporated into guiding sheath assembly (210) in place of control handle (216), and that may provide at least some, if not all, of the features and functionalities described above. Control handle (810) may be similar to control handle (216) described above, except as otherwise described below. In this regard, control handle (810) of the present example includes an elongate, generally cylindrical main body (812) similar to main body (224) described above, and a distal rotational control knob (not shown), such as distal rotational control knob (226) described above, mounted on a distal stem of main body (812). As shown, control handle (810) also includes a pair of shuttles (814a, 814b) similar to shuttles (232A, 232B) described above. A rotatable shaft (not shown), such as rotatable shaft (231) described above, is configured to drive longitudinal translation of first shuttle (814a) in response to rotation of the control knob; and a pinion (not shown), such as pinion (234) described above, couples shuttles (814a, 814b) to each other via respective teeth (not shown) of shuttles (814a, 814b) to thereby drive longitudinal translation of second shuttle (814b) in an opposite direction. Stops (also referred to as ferrules) (818a, 818b) similar to stops (289A, 289B) described above are affixed to the proximal ends of respective actuator elements (not shown), such as puller wires (230A, 230B) described above or any other suitable actuator elements (e.g., bands, rods, etc.), and are each positioned proximal of a proximal end (819a, 819b) of a corresponding shuttle (814a, 814b) so that each shuttle (814a, 814b) can push or otherwise act on the respective stop (818a, 818b) to draw the respective puller wire proximally when the respective shuttle (814a, 814b) is moved proximally from a neutral position (e.g., both shuttles (814a, 814b) in FIG. 11A) toward a proximal position (e.g., first shuttle (814a) in FIG. 11B, second shuttle (814b) in FIG. 11C); when a shuttle (814a, 814b) is moved distally from the proximal position (e.g., first shuttle (814a) in FIG. 11B, second shuttle (814b) in FIG. 11C) toward the neutral position (e.g., both shuttles (814a, 814b) in FIG. 11A), the respective stop (818a, 818b) may move distally with the shuttle (814a, 814b) as tension is relieved in the respective puller wire; when a shuttle (814a, 814b) is moved distally from the neutral position (e.g., both shuttles (814a, 814b) in FIG. 11A) toward a distal position (e.g., first shuttle (814a) in FIG. 11C, second shuttle (814b) in FIG. 11B), the proximal end (819a, 819b) of the shuttle (814a, 814b) comes out of contact with the respective stop (818a, 818b), releasing the respective puller wire from tension, such that the respective stop (818a, 818b) may remain stationary at its own neutral position. Thus, control handle (810) may provide bi-directional deflection of deflection section (214) of guiding sheath (212) in a manner similar to that described above in connection with control handle (216).

Control handle (810) of the present example further includes a deflection sensor assembly (820) configured to generate one or more signals indicative of an articulation state of guiding sheath (212). In the example shown, deflection sensor assembly (820) includes first and second longitudinally-adjacent proximity sensors in the form of reed switches (824a, 824b) fixedly secured to main body (812). In this regard, first shuttle (814a) includes a magnetic member in the form of an embedded permanent magnet (830); and the activation zones of reed switches (824a, 824b) each intersect with (e.g., are perpendicular to) the translation path of magnet (830) such that magnet (830) is configured to selectively trigger reed switches (824a, 824b) by selectively crossing the activation zones of reed switch (824a, 824b).

More particularly, magnet (830) of the present example is disposed at a predetermined position along a length of first shuttle (814a) such that magnet (830) is configured to be outside both of (e.g., in-between) the activation zones of reed switches (824a, 824b) when shuttles (814a, 814b) are even with each other in the longitudinal direction to place guiding sheath (212) in the neutral configuration; and such that magnet (830) is configured to trigger first reed switch (824a) and to be outside the activation zone of second reed switch (824b) when first shuttle (814a) is moved proximally and second shuttle (814b) is moved distally to place guiding sheath (212) in a deflected configuration in which guiding sheath is deflected in a first direction (e.g., rightward); and such that magnet (830) is configured to trigger second reed switch (824b) and to be outside the activation zone of first reed switch (824a) when first shuttle (814a) is moved distally and second shuttle (814b) is moved proximally to place guiding sheath (212) in a deflected configuration in which guiding sheath is deflected in a second direction (e.g., leftward). Therefore, in addition to indicating whether guiding sheath (212) is in a neutral or deflected configuration, the one or more signals generated by deflection sensor assembly (820) may indicate a direction of deflection when guiding sheath (212) is in a deflected configuration; and may be generated using relatively low current and without requiring physical contact between deflection sensor assembly (820) and either shuttle (814a, 814b). In some versions, magnet (830) may be oriented perpendicular to the longitudinal axis that shuttles (814a, 814b) translate along to improve the resolution of reed switches (824a, 824b).

Deflection sensor assembly (820) may be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assembly (820). In such versions, the processor of console (12) may be operable to process the articulation indicative signals from deflection sensor assembly (820) to thereby determine the current articulation state of guiding sheath (212). For instance, the processor of console (12) may be operable to determine that guiding sheath (212) is currently in the neutral configuration in response to not receiving a signal from deflection sensor assembly (820) (indicating that reed switches (824a, 824b) are currently not triggered by magnet (830)); and may be operable to determine that guiding sheath (212) is currently in a deflected configuration in which guiding sheath is deflected in the first direction (e.g., rightward) in response to receiving a signal from deflection sensor assembly (820) indicating that first reed switch (824a) is currently triggered by magnet (830) and that second reed switch (824b) is currently not triggered by magnet (830); and may be operable to determine that guiding sheath (212) is currently in a deflected configuration in which guiding sheath is deflected in the second direction (e.g., leftward) in response to receiving a signal from deflection sensor assembly (820) indicating that first reed switch (824a) is currently not triggered by magnet (830) and that second reed switch (824b) is currently triggered by magnet (830). The processor of console (12) may further be operable to provide a visual indication of the articulation state of guiding sheath (212) in real time via display (18), as described in greater detail below.

In an example of a method of use, guiding sheath (212) may initially be in the neutral configuration, such that magnet (830) is distal of and outside the activation zone of first reed switch (824a) and such that magnet (830) is proximal of and outside the activation zone of second reed switch (824b) as shown in FIG. 11A, and the operator may observe a real time indication that guiding sheath (212) is in the neutral configuration on display (18). Guiding sheath (212) may subsequently be articulated to a first deflected configuration in which guiding sheath (212) is deflected in the first direction (e.g., rightward), such that magnet (830) triggers first reed switch (824a) and such that magnet (830) is proximal of and outside the activation zone of second reed switch (824b) as shown in FIG. 11B, and the operator may observe a real time indication that guiding sheath (212) is deflected in the first direction on display (18). In addition, or alternatively, guiding sheath (212) may be articulated to a second deflected configuration in which guiding sheath (212) is deflected in the second direction (e.g., leftward), such that magnet (830) triggers second reed switch (824b) and such that magnet (830) is distal of and outside the activation zone of first reed switch (824a) as shown in FIG. 11C, and the operator may observe a real time indication that guiding sheath (212) is deflected in the second direction on display (18). In some instances, guiding sheath (212) may eventually be returned to the neutral configuration, such that magnet (830) is distal of and outside the activation zone of first reed switch (824a) and such that magnet (830) is proximal of and outside the activation zone of second reed switch (824b) as shown in FIG. 11A, and the operator may observe a real time indication that guiding sheath (212) is in the neutral configuration on display (18), before retracting guiding sheath (212) from the patient's heart (H).

### G. Example of Control Handle with Dual Stop Contact Sensors

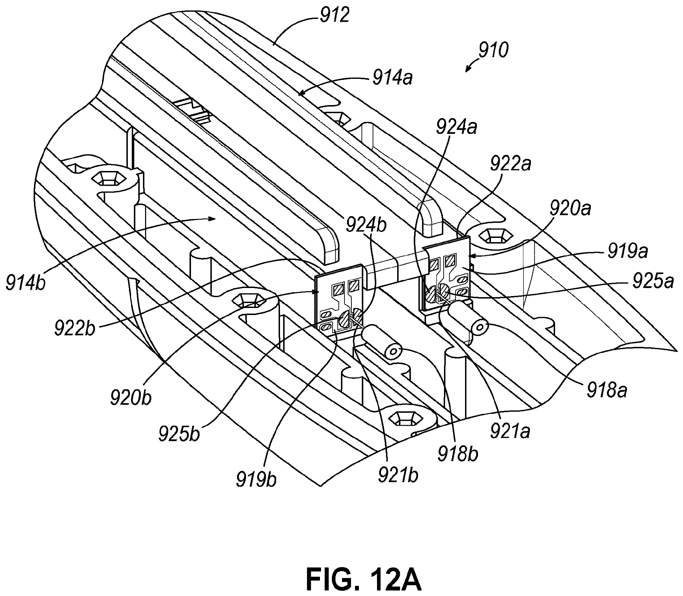

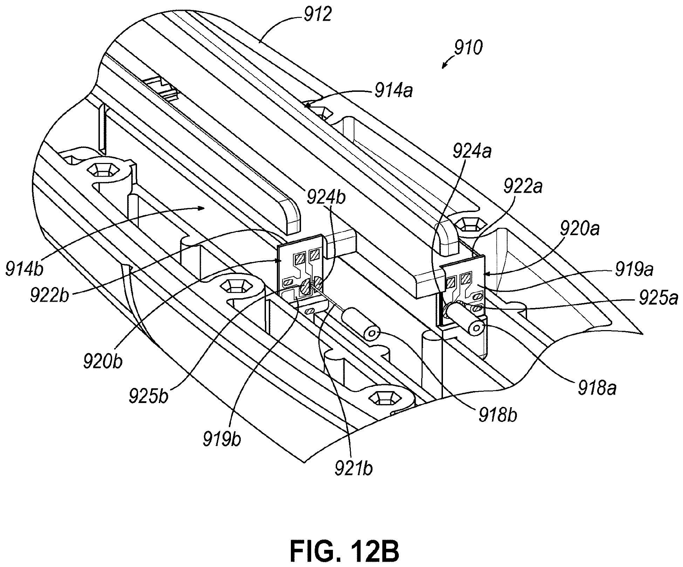

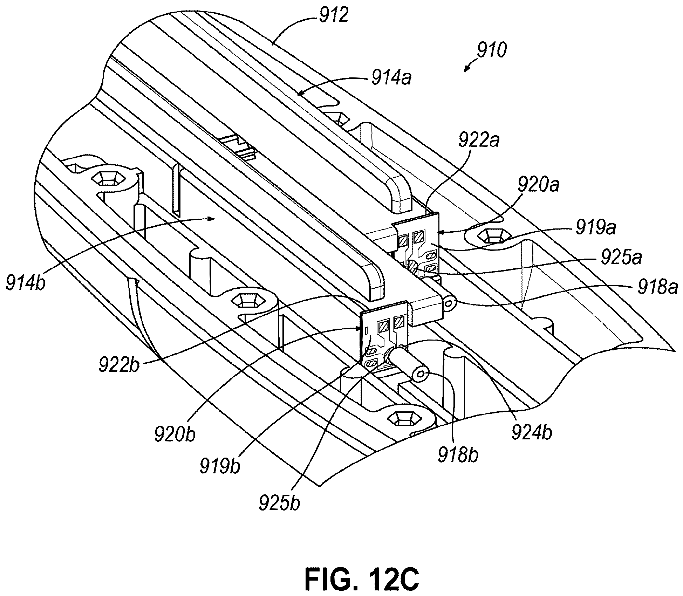

FIGS. 12A-12C show an example of a control handle (910) that may be incorporated into guiding sheath assembly (210) in place of control handle (216), and that may provide at least some, if not all, of the features and functionalities described above. Control handle (910) may be similar to control handle (216) described above, except as otherwise described below. In this regard, control handle (910) of the present example includes an elongate, generally cylindrical main body (912) similar to main body (224) described above, and a distal rotational control knob (not shown), such as distal rotational control knob (226) described above, mounted on a distal stem of main body (912). As shown, control handle (910) also includes a pair of shuttles (914a, 914b) similar to shuttles (232A, 232B) described above. A rotatable shaft (not shown), such as rotatable shaft (231) described above, is configured to drive longitudinal translation of first shuttle (914a) in response to rotation of the control knob; and a pinion (not shown), such as pinion (234) described above, couples shuttles (914a, 914b) to each other via respective teeth (not shown) of shuttles (914a, 914b) to thereby drive longitudinal translation of second shuttle (914b) in an opposite direction. Stops (also referred to as ferrules) (918a, 918b) similar to stops (289A, 289B) described above are affixed to the proximal ends of respective actuator elements in the form of puller wires (921a, 921b) similar to puller wires (230A, 230B) described above, and are each positioned proximal of a proximal end (919a, 919b) of a corresponding shuttle (914a, 914b) so that each shuttle (914a, 914b) can push or otherwise act on the respective stop (918a, 918b) to draw the respective puller wire (921a, 921b) proximally when the respective shuttle (914a, 914b) is moved proximally from a neutral position (e.g., both shuttles (914a, 914b) in FIG. 12A) toward a proximal position (e.g., first shuttle (914a) in FIG. 12B, second shuttle (914b) in FIG. 12C); when a shuttle (914a, 914b) is moved distally from the proximal position (e.g., first shuttle (914a) in FIG. 12B, second shuttle (914b) in FIG. 12C) toward the neutral position (e.g., both shuttles (914a, 914b) in FIG. 12A), the respective stop (918a, 918b) may move distally with the shuttle (914a, 914b) as tension is relieved in the respective puller wire (921a, 921b) until the respective stop (918a, 918b) reaches its own neutral position at which the proximal end (919a, 919b) of the shuttle (914a, 914b) comes out of contact with the respective stop (918a, 918b), releasing the respective puller wire (921a, 921b) from tension and thereby providing a small spatial gap between the proximal end (919a, 919b) of each shuttle (914a, 914b) and the respective stop (918a, 918b) such that there is no contact between the proximal end (919a, 919b) of each shuttle (914a, 914b) and the respective stop (918a, 918b) when in the neutral position; when a shuttle (914a, 914b) is moved distally from the neutral position (e.g., both shuttles (914a, 914b) in FIG. 12A) toward a distal position (e.g., first shuttle (914a) in FIG. 12C, second shuttle (914b) in FIG. 12B), the proximal end (919a, 919b) of the shuttle (914a, 914b) remains out of contact with the respective stop (918a, 918b), such that the respective stop (918a, 918b) may remain stationary at its own neutral position. Thus, control handle (910) may provide bi-directional deflection of deflection section (214) of guiding sheath (212) in a manner similar to that described above in connection with control handle (216).

Control handle (910) of the present example further includes first and second deflection sensor assemblies (920a, 920b) each configured to generate one or more signals indicative of an articulation state of guiding sheath (212). In the example shown, each deflection sensor assembly (920a, 920b) includes a printed circuit board (PCB) (922a, 922b) fixedly secured to the body of a respective shuttle (914a, 914b), such that a proximal surface of each PCB (922a, 922b) defines the proximal end (919a, 919b) of the respective shuttle (914a, 914b). Each PCB (922a, 922b) includes a corresponding pair of electrical contact members in the form of electrical contact traces (924a, 924b, 925a, 925b) that are spaced apart from each other about a corresponding opening for the corresponding puller wire (921a, 921b), such that the electrical contact traces (924a, 924b, 925a, 925b) of each pair are electrically isolated from each other on the respective PCB (922a, 922b) and are configured to be selectively electrically coupled to each other by the corresponding stop (918a, 918b). In this regard, each stop (918a, 918b) may comprise an electrically conductive and/or metallic material, such as stainless steel, such that each stop (918a, 918b) is configured to selectively electrically couple the corresponding pair of electrical contact traces (924a, 924b, 925a, 925b) by selectively contacting the corresponding pair of electrical contact traces (924a, 924b, 925a, 925b); and each electrical contact trace (924a, 924b, 925a, 925b) may comprise an electrically conductive and/or metallic material, such as copper.

More particularly, stops (918a, 918b) of the present example are configured to be out of contact with the corresponding pair of electrical contact traces (924a, 924b, 925a, 925b) such that the corresponding pair of electrical contact traces (924a, 924b, 925a, 925b) are electrically isolated from each other when shuttles (914a, 914b) are even with each other in the longitudinal direction to place guiding sheath (212) in the neutral configuration; and first stop (918a) is configured to contact and electrically couple the first pair of electrical contact traces (924a, 925a) and second stop (918b) is configured to be out of contact with the second pair of electrical contact traces (924b, 925b) such that the second pair of electrical contact traces (924b, 925b) are electrically isolated from each other when first shuttle (914a) is moved proximally and second shuttle (914b) is moved distally to place guiding sheath (212) in a deflected configuration in which guiding sheath is deflected in a first direction (e.g., rightward); and first stop (918a) is configured to be out of contact with the first pair of electrical contact traces (924a, 925a) such that the first pair of electrical contact traces (924a, 925a) are electrically isolated from each other and second stop (918b) is configured to contact and electrically couple the second pair of electrical contact traces (924b, 925b) when first shuttle (914a) is moved distally and second shuttle (914b) is moved proximally to place guiding sheath (212) in a deflected configuration in which guiding sheath is deflected in a second direction (e.g., leftward). Therefore, in addition to indicating whether guiding sheath (212) is in a neutral or deflected configuration, the one or more signals generated by deflection sensor assemblies (920a, 920b) may indicate a direction of deflection when guiding sheath (212) is in a deflected configuration.

Deflection sensor assemblies (920a, 920b) may each be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assemblies (920a, 920b). In such versions, the processor of console (12) may be operable to process the articulation indicative signals from deflection sensor assemblies (920a, 920b) to thereby determine the current articulation state of guiding sheath (212). For instance, the processor of console (12) may be operable to determine that guiding sheath (212) is currently in the neutral configuration in response to not receiving a signal from either deflection sensor assembly (920a, 920b) (indicating that each pair of electrical contact traces (924a, 924b, 925a, 925b) is currently not contacted by the respective stop (918a, 918b)); and may be operable to determine that guiding sheath (212) is currently in a deflected configuration in which guiding sheath is deflected in the first direction (e.g., rightward) in response to receiving a signal from first deflection sensor assembly (920a) indicating that first electrical contact traces (924a, 925a) are currently contacted and electrically coupled by first stop (918a) while not receiving a signal from second deflection sensor assembly (920b) (indicating that second electrical contact traces (924b, 925b) are currently not contacted by second stop (918b)); and may be operable to determine that guiding sheath (212) is currently in a deflected configuration in which guiding sheath is deflected in the second direction (e.g., leftward) in response to receiving a signal from second deflection sensor assembly (920b) indicating that second electrical contact traces (924b, 925b) are currently contacted and electrically coupled by second stop (918b) while not receiving a signal from first deflection sensor assembly (920a) (indicating that first electrical contact traces (924a, 925a) are currently not contacted by first stop (918a)). The processor of console (12) may further be operable to provide a visual indication of the articulation state of guiding sheath (212) in real time via display (18), as described in greater detail below.

In an example of a method of use, guiding sheath (212) may initially be in the neutral configuration, such that stops (918a, 918b) are each proximal of and not in contact with the corresponding pair of electrical contact traces (924a, 924b, 925a, 925b) as shown in FIG. 12A, and the operator may observe a real time indication that guiding sheath (212) is in the neutral configuration on display (18). Guiding sheath (212) may subsequently be articulated to a first deflected configuration in which guiding sheath (212) is deflected in the first direction (e.g., rightward), such that first stop (918a) contacts and electrically couples the first pair of electrical contact traces (924a, 925a) and such that second stop (918b) is proximal of and not in contact with the second pair of electrical contact traces (924b, 925b) as shown in FIG. 12B, and the operator may observe a real time indication that guiding sheath (212) is deflected in the first direction on display (18). In addition, or alternatively, guiding sheath (212) may be articulated to a second deflected configuration in which guiding sheath (212) is deflected in the second direction (e.g., leftward), such that second stop (918b) contacts and electrically couples the second pair of electrical contact traces (924b, 925b) and such that first stop (918a) is proximal of and not in contact with the first pair of electrical contact traces (924a, 925a) as shown in FIG. 12C, and the operator may observe a real time indication that guiding sheath (212) is deflected in the second direction on display (18). In some instances, guiding sheath (212) may eventually be returned to the neutral configuration, such that stops (918a, 918b) are each proximal of and not in contact with the corresponding pair of electrical contact traces (924a, 924b, 925a, 925b) as shown in FIG. 12A, and the operator may observe a real time indication that guiding sheath (212) is in the neutral configuration on display (18), before retracting guiding sheath (212) from the patient's heart (H).

### H. Example of Control Handle with Flexible Sensor Assembly

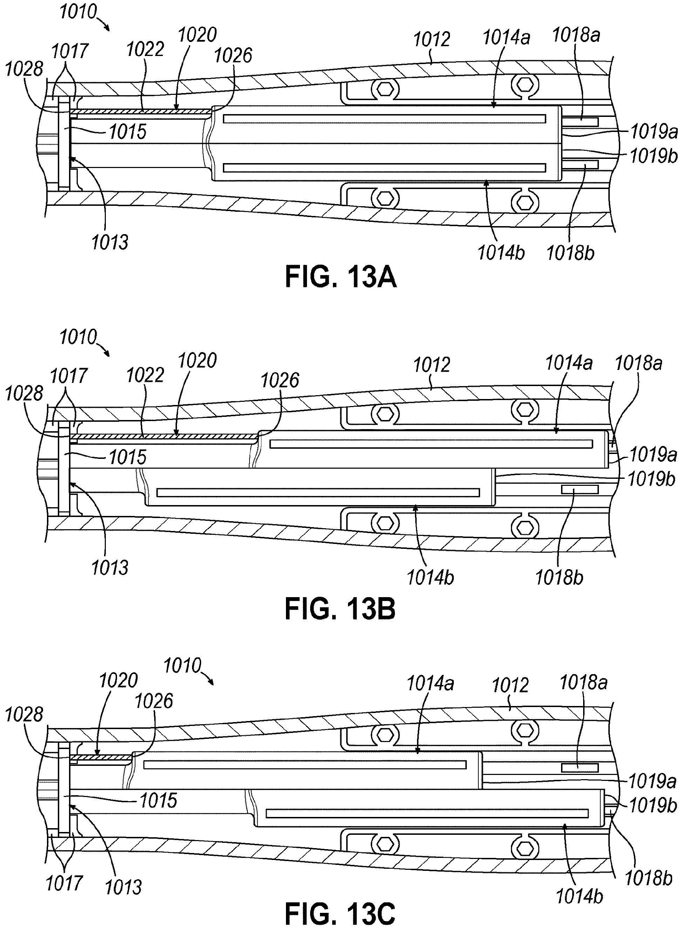

FIGS. 13A-13C show an example of a control handle (1010) that may be incorporated into guiding sheath assembly (210) in place of control handle (216), and that may provide at least some, if not all, of the features and functionalities described above. Control handle (1010) may be similar to control handle (216) described above, except as otherwise described below. In this regard, control handle (1010) of the present example includes an elongate, generally cylindrical main body (1012) similar to main body (224) described above, and a distal rotational control knob (not shown), such as distal rotational control knob (226) described above, mounted on a distal stem of main body (1012). As shown, control handle (1010) also includes a pair of shuttles (1014a, 1014b) similar to shuttles (232A, 232B) described above. A rotatable shaft (1013) similar to rotatable shaft (231) described above is connected and affixed at its proximal end to main body (1012) by a proximal outer circumferential lip (1015) that engages with an inner circumferential slot defined between circumferential flanges (1017), and is configured to drive longitudinal translation of first shuttle (1014a) in response to rotation of the control knob; and a pinion (not shown), such as pinion (234) described above, couples shuttles (1014a, 1014b) to each other via respective teeth (not shown) of shuttles (1014a, 1014b) to thereby drive longitudinal translation of second shuttle (1014b) in an opposite direction. Stops (also referred to as ferrules) (1018a, 1018b) similar to stops (289A, 289B) described above are affixed to the proximal ends of respective actuator elements (not shown), such as puller wires (230A, 230B) described above or any other suitable actuator elements (e.g., bands, rods, etc.), and are each positioned proximal of a proximal end (1019a, 1019b) of a corresponding shuttle (1014a, 1014b) so that each shuttle (1014a, 1014b) can push or otherwise act on the respective stop (1018a, 1018b) to draw the respective puller wire proximally when the respective shuttle (1014a, 1014b) is moved proximally from a neutral position (e.g., both shuttles (1014a, 1014b) in FIG. 13A) toward a proximal position (e.g., first shuttle (1014a) in FIG. 13B, second shuttle (1014b) in FIG. 13C); when a shuttle (1014a, 1014b) is moved distally from the proximal position (e.g., first shuttle (1014a) in FIG. 13B, second shuttle (1014b) in FIG. 13C) toward the neutral position (e.g., both shuttles (1014a, 1014b) in FIG. 13A), the respective stop (1018a, 1018b) may move distally with the shuttle (1014a, 1014b) as tension is relieved in the respective puller wire; when a shuttle (1014a, 1014b) is moved distally from the neutral position (e.g., both shuttles (1014a, 1014b) in FIG. 13A) toward a distal position (e.g., first shuttle (1014a) in FIG. 13C, second shuttle (1014b) in FIG. 13B), the proximal end (1019a, 1019b) of the shuttle (1014a, 1014b) comes out of contact with the respective stop (1018a, 1018b), releasing the respective puller wire from tension, such that the respective stop (1018a, 1018b) may remain stationary at its own neutral position. Thus, control handle (1010) may provide bi-directional deflection of deflection section (214) of guiding sheath (212) in a manner similar to that described above in connection with control handle (216).

Control handle (1010) of the present example further includes a deflection sensor assembly (1020) configured to generate one or more signals indicative of an articulation state of guiding sheath (212). In the example shown, deflection sensor assembly (1020) includes a flexible printed circuit board (PCB) (1022) with a variable length and having a proximal end (1026) fixedly secured to first shuttle (1014a) (e.g., to a distally-facing surface of first shuttle (1014a) that is proximal of shaft (1013)) and a distal end (1028) fixedly secured to main body (1012) (e.g., to a proximal one of flanges (1017)), such that flexible PCB (1022) is configured to flex (e.g., stretch and/or compress) between at least one elongated state (FIG. 13B), at least one shortened state (FIG. 13C), and an intermediate state (FIG. 13A). In this regard, flexible PCB (1022) may have at least one electrical trace (not shown) with a variable electrical resistance that varies based on the length of flexible PCB (1022) (e.g., based on a longitudinal distance between proximal and distal ends (1026, 1028)).

More particularly, flexible PCB (1022) of the present example is configured to be in the intermediate state such that the at least one electrical trace of flexible PCB (1022) has a first resistance when shuttles (1014a, 1014b) are even with each other in the longitudinal direction to place guiding sheath (212) in the neutral configuration; and flexible PCB (1022) is configured to be in the at least one elongated state such that the at least one electrical trace of flexible PCB (1022) has at least one second resistance greater than the first resistance when first shuttle (1014a) is moved proximally and second shuttle (1014b) is moved distally to place guiding sheath (212) in a deflected configuration in which guiding sheath is deflected in a first direction (e.g., rightward); and flexible PCB (1022) is configured to be in the at least one shortened state such that the at least one electrical trace of flexible PCB (1022) has at least one third resistance less than the first resistance when first shuttle (1014a) is moved distally and second shuttle (1014b) is moved proximally to place guiding sheath (212) in a deflected configuration in which guiding sheath is deflected in a second direction (e.g., leftward).

In some cases, flexible PCB (1022) may be configured to assume a plurality of elongated states, such as between the intermediate state shown in FIG. 13A and the elongated state shown in FIG. 13B, and the at least one second resistance of the at least one electrical trace of flexible PCB (1022) may include a plurality of second resistances that are different for each elongated state. Similarly, flexible PCB (1022) may be configured to assume a plurality of shortened states, such as between the intermediate state shown in FIG. 13A and the shortened state shown in FIG. 13C, and the at least one third resistance of the at least one electrical trace of flexible PCB (1022) may include a plurality of third resistances that are different for each elongated state. In such cases, the particular resistance of the at least one electrical trace of flexible PCB (1022) at a given moment may be indicative of the current length of flexible PCB (1022), which may be correlated to the longitudinal position of first shuttle (1014a) relative to main body (1012), and thus further correlated to a particular degree of deflection of guiding sheath (212). Therefore, in addition to indicating whether guiding sheath (212) is in a neutral or deflected configuration, and in addition to indicating a direction of deflection when guiding sheath (212) is in a deflected configuration, the one or more signals generated by deflection sensor assembly (1020) may indicate a particular degree of deflection when guiding sheath (212) is in a deflected configuration.

Deflection sensor assembly (1020) may be in operative communication (e.g., via a wired connection through an electrical connector, such as electrical connector (217) described above) with a guidance and drive system (not shown), such as guidance and drive system (10) described above, for sending one or more signals thereto. For example, first driver module (14) of console (12) of guidance and drive system (10) may be operable to receive articulation indicative signals from deflection sensor assembly (1020). In such versions, the processor of console (12) may be operable to process the articulation indicative signals from deflection sensor assembly (1020) to thereby determine the current articulation state of guiding sheath (212). For instance, the processor of console (12) may be operable to determine that guiding sheath (212) is currently in the neutral configuration in response to receiving a signal from deflection sensor assembly (1020) that is based on the first resistance of the at least one electrical trace of flexible PCB (1022), indicating that flexible PCB (1022) is in the intermediate state; and may be operable to determine that guiding sheath (212) is currently in a deflected configuration in which guiding sheath is deflected in the first direction (e.g., rightward) in response to receiving a signal from deflection sensor assembly (1020) that is based on the at least one second resistance of the at least one electrical trace of flexible PCB (1022), indicating that flexible PCB (1022) is in the at least one elongated state; and may be operable to determine that guiding sheath (212) is currently in a deflected configuration in which guiding sheath is deflected in the second direction (e.g., leftward) in response to receiving a signal from deflection sensor assembly (1020) that is based on the at least one third resistance of the at least one electrical trace of flexible PCB (1022), indicating that flexible PCB (1022) is in the at least one shortened state. In some cases, the processor of console (12) may be operable to determine the particular degree of deflection of guiding sheath (212) by correlating the at least one second resistance or the at least one third resistance of the at least one electrical trace of flexible PCB (1022) to the longitudinal position of first shuttle (1014a) relative to the main body (1012). The processor of console (12) may further be operable to provide a visual indication of the articulation state of guiding sheath (212) in real time via display (18), as described in greater detail below.

In an example of a method of use, guiding sheath (212) may initially be in the neutral configuration, such that flexible PCB (1022) is in the intermediate state as shown in FIG. 13A, and the operator may observe a real time indication that guiding sheath (212) is in the neutral configuration on display (18). Guiding sheath (212) may subsequently be articulated to a first deflected configuration in which guiding sheath (212) is deflected in the first direction (e.g., rightward), such that flexible PCB (1022) is in the elongated state as shown in FIG. 13B, and the operator may observe a real time indication that guiding sheath (212) is deflected in the first direction and/or a real time indication of the particular degree of deflection of guiding sheath (212) in the first direction on display (18). In addition, or alternatively, guiding sheath (212) may be articulated to a second deflected configuration in which guiding sheath (212) is deflected in the second direction (e.g., leftward), such that flexible PCB (1022) is in the shortened state as shown in FIG. 13C, and the operator may observe a real time indication that guiding sheath (212) is deflected in the second direction and/or a real time indication of the particular degree of deflection of guiding sheath (212) in the second direction on display (18). In some instances, guiding sheath (212) may eventually be returned to the neutral configuration, such that flexible PCB (1022) is in the intermediate state as shown in FIG. 13A, and the operator may observe a real time indication that guiding sheath (212) is in the neutral configuration on display (18), before retracting guiding sheath (212) from the patient's heart (H).

### V. Example of Graphical User Interface with Representation of Articulation State

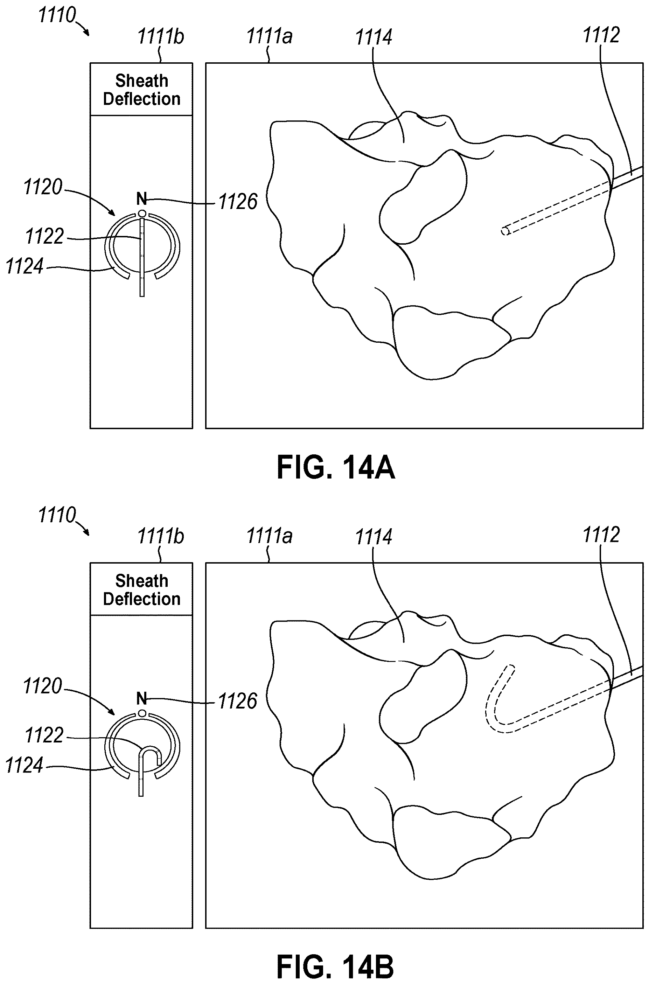

FIG. 14 shows an example of a graphical user interface (GUI) (1110) that may be rendered by a display of a guidance and drive system, such as by display (18) of guidance and drive system (10) described above, to allow the operator to observe a real time indication of the articulation state of guiding sheath (212) substantially simultaneously with observing the real time positioning of guiding sheath (212). GUI (1110) of the present example includes first and second windows (1111a, 1111b). The processor of console (12) may drive display (18) to superimpose a graphical representation (1112) of guiding sheath (212) on an image (1114) of the patient's heart (H) in first window (1111a). Image (1114) of heart (H) may be based on a set of preoperatively or intraoperatively obtained images (e.g., a CT or MRI scan, 3-D map, etc.). The views of heart (H) provided through display (18) may also change dynamically based on signals from the navigation sensor assembly of guiding sheath (212). Graphical representation (1112) of guiding sheath (212) may also move within image (1114) of heart (H) in first window (1111a) in real time as the physician moves the guiding sheath (212) within the patient (PA), thereby providing real-time visual feedback to the operator about the position of guiding sheath (212) within the patient (PA) as guiding sheath (212) moves within the patient (PA). In first window (1111a), display (18) may simultaneously visually indicate the locations of aberrant conductive tissue sites detected through EP mapping. The physician (PH) may thus view first window (1111a) of GUI (1110) on display (18) to observe the real time positioning of sheath (212) in relation to the mapped aberrant conductive tissue sites and in relation to image (1114) of heart (H) in the patient (PA).

The processor of console (12) may drive display (18) to render one or more graphical indicia (1120) in second window (1111b) indicating an articulation state of guiding sheath (212) based on one or more signals received by the processor from a deflection sensor assembly of a control handle for guiding sheath (212), such as any of deflection sensor assemblies (320, 420, 520, 620, 720, 820, 920a, 920b, 1020) of control handles (310, 410, 510, 610, 710, 810, 910, 1010) described above. In the example shown, graphical indicia (1120) includes a graphical representation (1122) of guiding sheath (212) overlayed on a generally circular scale (1124), such that graphical representation (1122) of guiding sheath (212) enters circular scale (1124) from a six o'clock position relative to circular scale (1124). Graphical indicia (1120) of the present example also includes an "N" marker (1126) at a twelve o'clock position relative to circular scale (1124).

When the processor determines that guiding sheath (212) is in the neutral configuration, the processor may drive display (18) to show graphical representation (1122) in a straight configuration such that graphical representation (1122) is aligned with the "N" marker (1126), as shown in FIG. 14A. When the processor determines that guiding sheath (212) is in a deflected configuration, the processor may drive display (18) to show graphical representation (1122) in a curved configuration such that graphical representation (1122) is deflected away from the "N" marker (1126), as shown in FIG. 14B. In some versions, such as those in which the one or more signals received by the processor from the deflection sensor assembly indicate a direction of deflection when guiding sheath (212) is in a deflected configuration, the processor may drive display (18) to show graphical representation (1122) in a curved configuration such that graphical representation (1122) is deflected away from the "N" marker (1126) in the same direction (e.g., rightward or leftward). In addition, or alternatively, in versions in which the one or more signals received by the processor from the deflection sensor assembly indicate a particular degree of deflection when guiding sheath (212) is in a deflected configuration, the processor may drive display (18) to show graphical representation (1122) in a curved configuration such that graphical representation (1122) is deflected away from the "N" marker (1126) to the same degree.

### VI. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A guiding sheath assembly, comprising: (a) an elongate shaft; (b) a control handle proximal of the elongate shaft, the control handle having a longitudinal axis, and including: (i) a control handle housing, and (ii) at least one shuttle configured for translation along the longitudinal axis; (c) at least one actuator element extending along at least one side of the elongate shaft and having a proximal end portion responsive to translation of the at least one shuttle in at least a proximal direction; and (d) at least one deflection sensor assembly secured to the control handle housing and configured to generate one or more signals indicative of an articulation state of the elongate shaft based on a longitudinal position of the at least one shuttle.

### Example 2

The guiding sheath assembly of Example 1, the at least one deflection sensor assembly including at least one tactile switch secured to the control handle housing, the at least one shuttle being configured to selectively actuate the at least one tactile switch.

### Example 3

The guiding sheath assembly of Example 2, the at least one tactile switch including first and second tactile switches, the at least one shuttle including first and second actuating members configured to selectively actuate the first and second tactile switches, respectively.

### Example 4

The guiding sheath assembly of any of Examples 1 through 3, the at least one deflection sensor assembly including at least one optical proximity sensor secured to the control handle housing, the at least one shuttle being configured to selectively trigger the at least one optical proximity sensor.

### Example 5

The guiding sheath assembly of Example 4, the at least one optical proximity sensor including first and second optical proximity sensors, the at least one shuttle including first and second shuttle configured to selectively trigger the first and second optical proximity sensors, respectively.

### Example 6

The guiding sheath assembly of any of Examples 1 through 5, the at least one deflection sensor assembly including at least one reed switch secured to the control handle housing, the at least one shuttle including at least one magnetic member configured to selectively trigger the at least one reed switch.

### Example 7

The guiding sheath assembly of Example 6, the at least one reed switch including first and second reed switches, the at least one magnetic member being configured to selectively trigger each of the first and second reed switches.

### Example 8

The guiding sheath assembly of any of Examples 1 through 7, the proximal end portion of the at least one actuator element being secured to an electrically conductive ferrule, the at least one deflection sensor assembly including at least one pair of electrically conductive contact members disposed on a proximal end of the at least one shuttle, the electrically conductive ferrule being configured to selectively electrically couple the at least one pair of electrically conductive contact members.

### Example 9

The guiding sheath assembly of any of Examples 1 through 8, the at least one deflection sensor assembly including at least one flexible sensor assembly having a variable resistance based on a length of the at least one flexible sensor assembly.

### Example 10

The guiding sheath assembly of any of Examples 1 through 9, the one or more signals being indicative of whether the elongate shaft is in a neutral configuration or at least one deflected configuration.

### Example 11

The guiding sheath assembly of Example 10, the one or more signals being indicative of a direction of deflection of the elongate shaft when the elongate shaft is in the at least one deflected configuration.

### Example 12

The guiding sheath assembly of any of Examples 10 through 11, the one or more signals being indicative of a degree of deflection of the elongate shaft when the elongate shaft is in the at least one deflected configuration.

### Example 13

The guiding sheath assembly of any of Examples 1 through 12, the at least one shuttle including: (A) a first shuttle configured for translation along the longitudinal axis in a first direction, and (B) a second shuttle configured for translation along the longitudinal axis in a second direction opposite the first direction in response to translation of the first shuttle in the first direction.

### Example 14

The guiding sheath assembly of Example 13, the at least one actuator element including: (i) a first actuator element extending along a first side of the elongate shaft and having a proximal end portion responsive to translation of the first shuttle in at least the proximal direction, and (ii) a second actuator element extending along a second side of the elongate shaft and having a proximal end portion responsive to translation of the second shuttle in at least the proximal direction.

### Example 15

The guiding sheath assembly of any of Examples 13 through 14, the first shuttle having a first plurality of teeth, the control handle further including a pinion in engagement with the first plurality of teeth, the pinion configured for rotation about an axis generally perpendicular to the longitudinal axis in response to translation of the first shuttle in the first direction, the second shuttle having a second plurality of teeth in engagement with the pinion, the second shuttle configured for translation along the longitudinal axis in the second direction in response to rotation of the pinion.

### Example 16

The guiding sheath assembly of any of Examples 1 through 15, the control handle including a rotatable shaft inside the control handle housing and configured for rotation about the longitudinal axis of the control handle, the at least one shuttle being configured for translation along the longitudinal axis in a first direction in response to rotation of the rotatable shaft.

### Example 17

The guiding sheath assembly of Example 16, the control handle including a control knob coupled for common translational movement and common rotational movement to the rotatable shaft.

### Example 18

The guiding sheath assembly of any of Examples 16 through 17, the at least one shuttle including first and second shuttles, the rotatable shaft comprising an inner passage, at least a portion of the first shuttle being configured to extend into the inner passage of the rotatable shaft, at least a portion of the second shuttle being configured to extend into the inner passage of the rotatable shaft.

### Example 19

A system, comprising: (a) the guiding sheath assembly of any of Examples 1 through 18; and (b) a processor in operative communication with the at least one deflection sensor assembly for receiving the one or more signals from the at least one deflection sensor assembly, the processor being configured to determine the articulation state of the elongate shaft based on the one or more signals.

### Example 20

A guiding sheath assembly, comprising: (a) an elongate shaft; (b) a control handle proximal of the elongate shaft, the control handle having a longitudinal axis, and including: (i) a control handle housing, (ii) a control knob configured for rotation about the longitudinal axis, (iii) a rotatable shaft inside the control handle housing and configured for rotation about the longitudinal axis in response to rotation of the control knob, the rotatable shaft being coupled for common translational movement and common rotational movement to the control knob, (iv) a first shuttle configured for translation along the longitudinal axis in a first direction in response to rotation of the rotatable shaft, and (v) a second shuttle configured for translation along the longitudinal axis in a second direction opposite to the first direction in response to translation of the first shuttle in the first direction; (c) a first puller wire extending along a first side of the elongated shaft and having a proximal end portion responsive to at least translation of the first shuttle in a proximal direction; (d) a second puller wire extending along a second side of the elongated shaft and having a proximal end portion responsive to at least translation of the second shuttle in the proximal direction; and (e) at least one proximity sensor secured to the control handle housing and configured to generate one or more signals indicative of an articulation state of the elongate shaft based on a longitudinal position of at least one of the first or second shuttles.

### Example 21

The guiding sheath assembly of Example 20, the at least one proximity sensor including at least one of an optical proximity sensor or a reed switch.

### Example 22

The guiding sheath assembly of any of Examples 20 through 21, the at least one proximity sensor including first and second proximity sensors, the first and second shuttles being configured to selectively trigger the first and second proximity sensors, respectively.

### Example 23

The guiding sheath assembly of any of Examples 20 through 22, the at least one proximity sensor including first and second proximity sensors, one of the first or second shuttles being configured to selectively trigger each of the first and second proximity sensors.

### Example 24

A control handle for use in controlling deflection of a medical guiding sheath shaft, the control handle having a longitudinal axis, and including: (a) a control handle housing; (b) a control knob configured for rotation about the longitudinal axis; (c) a rotatable shaft inside the control handle housing and configured for rotation about the longitudinal axis in response to rotation of the control knob, the rotatable shaft being coupled for common translational movement and common rotational movement to the control knob; (d) a first shuttle configured for translation along the longitudinal axis in a first direction in response to rotation of the rotatable shaft; (e) a second shuttle configured for translation along the longitudinal axis in a second direction opposite to the first direction in response to translation of the first shuttle in the first direction, the first and second shuttles being configured to respectively act on first and second puller wires extending along the medical guiding sheath shaft; and (f) at least one deflection sensor assembly secured to the control handle housing and configured to generate one or more signals indicative of an articulation state of the medical guiding sheath shaft based on a longitudinal position of at least one of the first or second shuttles.

### VII. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

### Aspects of the invention:

1. A guiding sheath assembly, comprising:
   (a) an elongate shaft;
   (b) a control handle proximal of the elongate shaft, the control handle having a longitudinal axis, and including:
      (i) a control handle housing,
      (ii) a control knob configured for rotation about the longitudinal axis,
      (iii) a rotatable shaft inside the control handle housing and configured for rotation about the longitudinal axis in response to rotation of the control knob, the rotatable shaft being coupled for common translational movement and common rotational movement to the control knob,
      (iv) a first shuttle configured for translation along the longitudinal axis in a first direction in response to rotation of the rotatable shaft, and
      (v) a second shuttle configured for translation along the longitudinal axis in a second direction opposite to the first direction in response to translation of the first shuttle in the first direction;
   (c) a first puller wire extending along a first side of the elongated shaft and having a proximal end portion responsive to at least translation of the first shuttle in a proximal direction;
   (d) a second puller wire extending along a second side of the elongated shaft and having a proximal end portion responsive to at least translation of the second shuttle in the proximal direction; and
   (e) at least one proximity sensor secured to the control handle housing and configured to generate one or more signals indicative of an articulation state of the elongate shaft based on a longitudinal position of at least one of the first or second shuttles.
2. The guiding sheath assembly of aspect 1, the at least one proximity sensor including at least one of an optical proximity sensor or a reed switch.
3. The guiding sheath assembly of aspect 1, the at least one proximity sensor including first and second proximity sensors, the first and second shuttles being configured to selectively trigger the first and second proximity sensors, respectively.
4. The guiding sheath assembly of aspect 1, the at least one proximity sensor including first and second proximity sensors, one of the first or second shuttles being configured to selectively trigger each of the first and second proximity sensors.
5. A control handle for use in controlling deflection of a medical guiding sheath shaft, the control handle having a longitudinal axis, and including:
   (a) a control handle housing;
   (b) a control knob configured for rotation about the longitudinal axis;
   (c) a rotatable shaft inside the control handle housing and configured for rotation about the longitudinal axis in response to rotation of the control knob, the rotatable shaft being coupled for common translational movement and common rotational movement to the control knob;
   (d) a first shuttle configured for translation along the longitudinal axis in a first direction in response to rotation of the rotatable shaft;
   (e) a second shuttle configured for translation along the longitudinal axis in a second direction opposite to the first direction in response to translation of the first shuttle in the first direction, the first and second shuttles being configured to respectively act on first and second puller wires extending along the medical guiding sheath shaft; and
   (f) at least one deflection sensor assembly secured to the control handle housing and configured to generate one or more signals indicative of an articulation state of the medical guiding sheath shaft based on a longitudinal position of at least one of the first or second shuttles.

## Claims

1. A guiding sheath assembly, comprising:
(a) an elongate shaft;
(b) a control handle proximal of the elongate shaft, the control handle having a longitudinal axis, and including:
(i) a control handle housing, and
(ii) at least one shuttle configured for translation along the longitudinal axis;
(c) at least one actuator element extending along at least one side of the elongate shaft and having a proximal end portion responsive to translation of the at least one shuttle in at least a proximal direction; and
(d) at least one deflection sensor assembly secured to the control handle housing and configured to generate one or more signals indicative of an articulation state of the elongate shaft based on a longitudinal position of the at least one shuttle.

2. The guiding sheath assembly of claim 1, the at least one deflection sensor assembly including at least one tactile switch secured to the control handle housing, the at least one shuttle being configured to selectively actuate the at least one tactile switch.

3. The guiding sheath assembly of claim 2, the at least one tactile switch including first and second tactile switches, the at least one shuttle including first and second actuating members configured to selectively actuate the first and second tactile switches, respectively.

4. The guiding sheath assembly of any preceding claim, the at least one deflection sensor assembly including at least one optical proximity sensor secured to the control handle housing, the at least one shuttle being configured to selectively trigger the at least one optical proximity sensor.

5. The guiding sheath assembly of claim 4, the at least one optical proximity sensor including first and second optical proximity sensors, the at least one shuttle including first and second shuttle configured to selectively trigger the first and second optical proximity sensors, respectively.

6. The guiding sheath assembly of any preceding claim, the at least one deflection sensor assembly including at least one reed switch secured to the control handle housing, the at least one shuttle including at least one magnetic member configured to selectively trigger the at least one reed switch.

7. The guiding sheath assembly of claim 6, the at least one reed switch including first and second reed switches, the at least one magnetic member being configured to selectively trigger each of the first and second reed switches.

8. The guiding sheath assembly of any preceding claim, the proximal end portion of the at least one actuator element being secured to an electrically conductive ferrule, the at least one deflection sensor assembly including at least one pair of electrically conductive contact members disposed on a proximal end of the at least one shuttle, the electrically conductive ferrule being configured to selectively electrically couple the at least one pair of electrically conductive contact members.

9. The guiding sheath assembly of any preceding claim, the at least one deflection sensor assembly including at least one flexible sensor assembly having a variable resistance based on a length of the at least one flexible sensor assembly.

10. The guiding sheath assembly of any preceding claim, the one or more signals being indicative of whether the elongate shaft is in a neutral configuration or at least one deflected configuration.

11. The guiding sheath assembly of claim 10, the one or more signals being indicative of a direction of deflection of the elongate shaft when the elongate shaft is in the at least one deflected configuration.

12. The guiding sheath assembly of claim 10 or claim 11, the one or more signals being indicative of a degree of deflection of the elongate shaft when the elongate shaft is in the at least one deflected configuration.

13. The guiding sheath assembly of any preceding claim, the at least one shuttle including:
(A) a first shuttle configured for translation along the longitudinal axis in a first direction, and
(B) a second shuttle configured for translation along the longitudinal axis in a second direction opposite the first direction in response to translation of the first shuttle in the first direction.

14. The guiding sheath assembly of claim 13, the at least one actuator element including:
(i) a first actuator element extending along a first side of the elongate shaft and having a proximal end portion responsive to translation of the first shuttle in at least the proximal direction, and
(ii) a second actuator element extending along a second side of the elongate shaft and having a proximal end portion responsive to translation of the second shuttle in at least the proximal direction.

15. A system, comprising:
(a) the guiding sheath assembly of claim 1; and
(b) a processor in operative communication with the at least one deflection sensor assembly for receiving the one or more signals from the at least one deflection sensor assembly, the processor being configured to determine the articulation state of the elongate shaft based on the one or more signals.
